(19)

Europäisches
Patentamt
European
Patent Office
Office européen
des brevets

(11) **EP 2 660 324 A1**

(12) **EUROPEAN PATENT APPLICATION**

(43) Date of publication:
**06.11.2013 Bulletin 2013/45**

(51) Int Cl.:
**C12N 15/82** $^{(2006.01)}$

(21) Application number: **12003136.4**

(22) Date of filing: **02.05.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Algenics**
**44800 Saint Herblain (FR)**

(72) Inventors:
• **Carlier, Aude**
**44000 Nantes (FR)**

• **Michel, Rémy**
**44100 Nantes (FR)**
• **Lejeune, Alexandre**
**44420 La Chapelle Sur Erdre (FR)**

(74) Representative: **Barbot, Willy**
**SIMODORO**
**Parc Cézanne II, Bât. G**
**290 Avenue Galilée**
**13857 Aix en Provence Cedex 3 (FR)**

(54) **Production of secreted therapeutic antibodies in microalgae**

(57)     The invention relates to a transformed microalga comprising a nucleic acid sequence operatively linked to a promoter, wherein said nucleic acid sequence encodes an amino acid sequence comprising (i) an heterologous signal peptide; and (ii) a therapeutic antibody, a functional fragment or a derivative thereof, said transformed microalga expressing the therapeutic antibody, functional fragment or derivative thereof secreted in the extracellular media and said microalga being selected among green algae except Volvocales, and among red algae, chromalveolates, and euglenids. Preferably, said therapeutic antibody, functional fragment or derivative thereof has an increased antibody-dependant cell-mediated cytotoxicity (ADCC) and a low fucose content. The present invention also relates to a method for producing said therapeutic antibody, a functional fragment or a derivative thereof, to the use of a transformed microalga according to the invention for the production and the secretion of a therapeutic antibody, a functional fragment or a derivative thereof in the extracellular medium, to a therapeutic antibody, a functional fragment or a derivative thereof produced and secreted in the extracellular medium of microalgae by a method according to the invention and to a pharmaceutical composition comprising said therapeutic antibody, functional fragment or derivative thereof.

EP 2 660 324 A1

SEQUENCE LISTING

```
<110>  ALGENICS
       CARLIER, Aude
       MICHEL, Rémy
       LEJEUNE, Alexandre

<120>  PRODUCTION OF SECRETED THERAPEUTIC ANTIBODIES IN MICROALGAE

<130>  ALG-B-0004 EP1

<160>  20

<170>  PatentIn version 3.5

<210>  1
<211>  230
<212>  PRT
<213>  homo sapiens

<400>  1

Met Gly Val Lys Val Leu Phe Ala Leu Ile Cys Ile Ala Val Ala Glu
1               5                   10                  15

Ala Asp Ile Leu Leu Thr Gln Ser Pro Val Ile Leu Ser Val Ser Pro
            20                  25                  30

Gly Glu Arg Val Ser Phe Ser Cys Arg Ala Ser Gln Ser Ile Gly Thr
            35                  40                  45

Asn Ile His Trp Tyr Gln Gln Arg Thr Asn Gly Ser Pro Arg Leu Leu
        50                  55                  60

Ile Lys Tyr Ala Ser Glu Ser Ile Ser Gly Ile Pro Ser Arg Phe Ser
65                  70                  75                  80

Gly Ser Gly Ser Gly Thr Asp Phe Thr Leu Ser Ile Asn Ser Val Glu
                85                  90                  95

Ser Glu Asp Ile Ala Asp Tyr Tyr Cys Gln Gln Asn Asn Asn Trp Pro
                100                 105                 110

Thr Thr Phe Gly Ala Gly Thr Lys Leu Glu Leu Lys Arg Thr Val Ala
        115                 120                 125

Ala Pro Ser Val Phe Ile Phe Pro Pro Ser Asp Glu Gln Leu Lys Ser
        130                 135                 140

Gly Thr Ala Ser Val Val Cys Leu Leu Asn Asn Phe Tyr Pro Arg Glu
145                 150                 155                 160

Ala Lys Val Gln Trp Lys Val Asp Asn Ala Leu Gln Ser Gly Asn Ser
                165                 170                 175

Gln Glu Ser Val Thr Glu Gln Asp Ser Lys Asp Ser Thr Tyr Ser Leu
                180                 185                 190
```

```
Ser Ser Thr Leu Thr Leu Ser Lys Ala Asp Tyr Glu Lys His Lys Val
        195             200             205

Tyr Ala Cys Glu Val Thr His Gln Gly Leu Ser Ser Pro Val Thr Lys
    210             215             220

Ser Phe Asn Arg Gly Ala
225             230


<210>  2
<211>  469
<212>  PRT
<213>  homo sapiens

<400>  2

Met Gly Val Lys Val Leu Phe Ala Leu Ile Cys Ile Ala Val Ala Glu
1               5           10              15

Ala Gln Val Gln Leu Lys Gln Ser Gly Pro Gly Leu Val Gln Pro Ser
            20              25              30

Gln Ser Leu Ser Ile Thr Cys Thr Val Ser Gly Phe Ser Leu Thr Asn
        35              40              45

Tyr Gly Val His Trp Val Arg Gln Ser Pro Gly Lys Gly Leu Glu Trp
    50              55              60

Leu Gly Val Ile Trp Ser Gly Gly Asn Thr Asp Tyr Asn Thr Pro Phe
65              70              75              80

Thr Ser Arg Leu Ser Ile Asn Lys Asp Asn Ser Lys Ser Gln Val Phe
            85              90              95

Phe Lys Met Asn Ser Leu Gln Ser Asn Asp Thr Ala Ile Tyr Tyr Cys
        100             105             110

Ala Arg Ala Leu Thr Tyr Tyr Asp Tyr Glu Phe Ala Tyr Trp Gly Gln
        115             120             125

Gly Thr Leu Val Thr Val Ser Ala Ala Ser Thr Lys Gly Pro Ser Val
    130             135             140

Phe Pro Leu Ala Pro Ser Ser Lys Ser Thr Ser Gly Gly Thr Ala Ala
145             150             155             160

Leu Gly Cys Leu Val Lys Asp Tyr Phe Pro Glu Pro Val Thr Val Ser
            165             170             175

Trp Asn Ser Gly Ala Leu Thr Ser Gly Val His Thr Phe Pro Ala Val
            180             185             190

Leu Gln Ser Ser Gly Leu Tyr Ser Leu Ser Ser Val Val Thr Val Pro
        195             200             205
```

```
Ser Ser Ser Leu Gly Thr Gln Thr Tyr Ile Cys Asn Val Asn His Lys
    210                 215                 220

Pro Ser Asn Thr Lys Val Asp Lys Arg Val Glu Pro Lys Ser Pro Lys
225             230                 235                     240

Ser Cys Asp Lys Thr His Thr Cys Pro Pro Cys Pro Ala Pro Glu Leu
                245                 250                 255

Leu Gly Gly Pro Ser Val Phe Leu Phe Pro Pro Lys Pro Lys Asp Thr
            260                 265                 270

Leu Met Ile Ser Arg Thr Pro Glu Val Thr Cys Val Val Val Asp Val
        275                 280                 285

Ser His Glu Asp Pro Glu Val Lys Phe Asn Trp Tyr Val Asp Gly Val
    290                 295                 300

Glu Val His Asn Ala Lys Thr Lys Pro Arg Glu Glu Gln Tyr Asn Ser
305                 310                 315                 320

Thr Tyr Arg Val Val Ser Val Leu Thr Val Leu His Gln Asp Trp Leu
            325                 330                 335

Asn Gly Lys Glu Tyr Lys Cys Lys Val Ser Asn Lys Ala Leu Pro Ala
        340                 345                 350

Pro Ile Glu Lys Thr Ile Ser Lys Ala Lys Gly Gln Pro Arg Glu Pro
        355                 360                 365

Gln Val Tyr Thr Leu Pro Pro Ser Arg Asp Glu Leu Thr Lys Asn Gln
    370                 375                 380

Val Ser Leu Thr Cys Leu Val Lys Gly Phe Tyr Pro Ser Asp Ile Ala
385             390                 395                     400

Val Glu Trp Glu Ser Asn Gly Gln Pro Glu Asn Asn Tyr Lys Thr Thr
            405                 410                     415

Pro Pro Val Leu Asp Ser Asp Gly Ser Phe Phe Leu Tyr Ser Lys Leu
            420                 425                 430

Thr Val Asp Lys Ser Arg Trp Gln Gln Gly Asn Val Phe Ser Cys Ser
        435                 440                 445

Val Met His Glu Ala Leu His Asn His Tyr Thr Gln Lys Ser Leu Ser
    450                 455                 460

Leu Ser Pro Gly Lys
465

<210>  3
```

```
<211>   19
<212>   DNA
<213>   Artificial sequence

<220>
<223>   primers used for the PCR amplification of the light chain

<400>   3
taccaacagc gaacgaacg                                                    19


<210>   4
<211>   19
<212>   DNA
<213>   Artificial sequence

<220>
<223>   primers used for the PCR amplification of the light chain

<400>   4
gtcgaccttc cattggacc                                                    19


<210>   5
<211>   19
<212>   DNA
<213>   Artificial sequence

<220>
<223>   primers used for the PCR amplification of the heavy chain

<400>   5
caaggacaac tcgaagtcg                                                    19


<210>   6
<211>   19
<212>   DNA
<213>   Artificial sequence

<220>
<223>   primers used for the PCR amplification of the heavy chain

<400>   6
cggttcgact cgcttgtcg                                                    19


<210>   7
<211>   235
<212>   PRT
<213>   homo sapiens

<400>   7
```

Met Asp Phe Gln Val Gln Ile Ile Ser Phe Leu Leu Ile Ser Ala Ser
1               5                   10                  15

Val Ile Met Ser Arg Gly Gln Ile Val Leu Ser Gln Ser Pro Ala Ile
            20                  25                  30

Leu Ser Ala Ser Pro Gly Glu Lys Val Thr Met Thr Cys Arg Ala Ser
            35                  40                  45

Ser Ser Val Ser Tyr Ile His Trp Phe Gln Gln Lys Pro Gly Ser Ser
        50                  55                  60

```
Pro Lys Pro Trp Ile Tyr Ala Thr Ser Asn Leu Ala Ser Gly Val Pro
65              70              75              80

Val Arg Phe Ser Gly Ser Gly Ser Gly Thr Ser Tyr Ser Leu Thr Ile
            85              90              95

Ser Arg Val Glu Ala Glu Asp Ala Ala Thr Tyr Tyr Cys Gln Gln Trp
            100             105             110

Thr Ser Asn Pro Pro Thr Phe Gly Gly Gly Thr Lys Leu Glu Ile Lys
        115             120             125

Arg Thr Val Ala Ala Pro Ser Val Phe Ile Phe Pro Pro Ser Asp Glu
    130             135             140

Gln Leu Lys Ser Gly Thr Ala Ser Val Val Cys Leu Leu Asn Asn Phe
145             150             155             160

Tyr Pro Arg Glu Ala Lys Val Gln Trp Lys Val Asp Asn Ala Leu Gln
            165             170             175

Ser Gly Asn Ser Gln Glu Ser Val Thr Glu Gln Asp Ser Lys Asp Ser
            180             185             190

Thr Tyr Ser Leu Ser Ser Thr Leu Thr Leu Ser Lys Ala Asp Tyr Glu
        195             200             205

Lys His Lys Val Tyr Ala Cys Glu Val Thr His Gln Gly Leu Ser Ser
    210             215             220

Pro Val Thr Lys Ser Phe Asn Arg Gly Glu Cys
225             230             235

<210>   8
<211>   470
<212>   PRT
<213>   homo sapiens

<400>   8

Met Gly Trp Ser Leu Ile Leu Leu Phe Leu Val Ala Val Ala Thr Arg
1               5               10              15

Val Leu Ser Gln Val Gln Leu Gln Gln Pro Gly Ala Glu Leu Val Lys
            20              25              30

Pro Gly Ala Ser Val Lys Met Ser Cys Lys Ala Ser Gly Tyr Thr Phe
        35              40              45

Thr Ser Tyr Asn Met His Trp Val Lys Gln Thr Pro Gly Arg Gly Leu
    50              55              60

Glu Trp Ile Gly Ala Ile Tyr Pro Gly Asn Gly Asp Thr Ser Tyr Asn
65              70              75              80
```

Gln Lys Phe Lys Gly Lys Ala Thr Leu Thr Ala Asp Lys Ser Ser Ser
                85                  90              95

Thr Ala Tyr Met Gln Leu Ser Ser Leu Thr Ser Glu Asp Ser Ala Val
            100             105             110

Tyr Tyr Cys Ala Arg Ser Thr Tyr Tyr Gly Gly Asp Trp Tyr Phe Asn
        115             120             125

Val Trp Gly Ala Gly Thr Thr Val Thr Val Ser Ala Ala Ser Thr Lys
    130             135             140

Gly Pro Ser Val Phe Pro Leu Ala Pro Ser Ser Lys Ser Thr Ser Gly
145             150             155             160

Gly Thr Ala Ala Leu Gly Cys Leu Val Lys Asp Tyr Phe Pro Glu Pro
            165             170             175

Val Thr Val Ser Trp Asn Ser Gly Ala Leu Thr Ser Gly Val His Thr
            180             185             190

Phe Pro Ala Val Leu Gln Ser Ser Gly Leu Tyr Ser Leu Ser Ser Val
        195             200             205

Val Thr Val Pro Ser Ser Ser Leu Gly Thr Gln Thr Tyr Ile Cys Asn
    210             215             220

Val Asn His Lys Pro Ser Asn Thr Lys Val Asp Lys Lys Ala Glu Pro
225             230             235             240

Lys Ser Cys Asp Lys Thr His Thr Cys Pro Pro Cys Pro Ala Pro Glu
            245             250             255

Leu Leu Gly Gly Pro Ser Val Phe Leu Phe Pro Pro Lys Pro Lys Asp
            260             265             270

Thr Leu Met Ile Ser Arg Thr Pro Glu Val Thr Cys Val Val Val Asp
            275             280             285

Val Ser His Glu Asp Pro Glu Val Lys Phe Asn Trp Tyr Val Asp Gly
    290             295             300

Val Glu Val His Asn Ala Lys Thr Lys Pro Arg Glu Glu Gln Tyr Asn
305             310             315             320

Ser Thr Tyr Arg Val Val Ser Val Leu Thr Val Leu His Gln Asp Trp
            325             330             335

Leu Asn Gly Lys Glu Tyr Lys Cys Lys Val Ser Asn Lys Ala Leu Pro
            340             345             350

Ala Pro Ile Glu Lys Thr Ile Ser Lys Ala Lys Gly Gln Pro Arg Glu

                    355                    360                    365

Pro Gln Val Tyr Thr Leu Pro Pro Ser Arg Asp Glu Leu Thr Lys Asn
    370                 375                 380

Gln Val Ser Leu Thr Cys Leu Val Lys Gly Phe Tyr Pro Ser Asp Ile
385                 390                 395                 400

Ala Val Glu Trp Glu Ser Asn Gly Gln Pro Glu Asn Asn Tyr Lys Thr
            405                 410                 415

Thr Pro Pro Val Leu Asp Ser Asp Gly Ser Phe Phe Leu Tyr Ser Lys
            420                 425                 430

Leu Thr Val Asp Lys Ser Arg Trp Gln Gln Gly Asn Val Phe Ser Cys
        435                 440                 445

Ser Val Met His Glu Ala Leu His Asn His Tyr Thr Gln Lys Ser Leu
    450                 455                 460

Ser Leu Ser Pro Gly Lys
465                 470

<210> 9
<211> 107
<212> PRT
<213> homo sapiens

<400> 9

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
            35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
            85                  90                  95

Thr Phe Gly Gln Gly Thr Lys Val Glu Ile Lys
            100                 105

<210> 10
<211> 120
<212> PRT
<213> homo sapiens

<400> 10

Glu Val Gln Leu Val Glu Ser Gly Gly Gly Leu Val Gln Pro Gly Gly
1               5               10              15

Ser Leu Arg Leu Ser Cys Ala Ala Ser Gly Phe Asn Ile Lys Asp Thr
            20              25              30

Tyr Ile His Trp Val Arg Gln Ala Pro Gly Lys Gly Leu Glu Trp Val
        35              40              45

Ala Arg Ile Tyr Pro Thr Asn Gly Tyr Thr Arg Tyr Ala Asp Ser Val
        50              55              60

Lys Gly Arg Phe Thr Ile Ser Ala Asp Thr Ser Lys Asn Thr Ala Tyr
65              70              75              80

Leu Gln Met Asn Ser Leu Arg Ala Glu Asp Thr Ala Val Tyr Tyr Cys
            85              90              95

Ser Arg Trp Gly Gly Asp Gly Phe Tyr Ala Met Asp Tyr Trp Gly Gln
            100             105             110

Gly Thr Leu Val Thr Val Ser Ser
        115             120

<210> 11
<211> 106
<212> PRT
<213> homo sapiens

<400> 11

Asp Ile Gln Met Thr Gln Ser Pro Ser Thr Leu Ser Ala Ser Val Gly
1               5               10              15

Asp Arg Val Thr Ile Thr Cys Lys Cys Gln Leu Ser Val Gly Tyr Met
            20              25              30

His Trp Tyr Gln Gln Lys Pro Gly Lys Ala Pro Lys Leu Leu Ile Tyr
        35              40              45

Asp Thr Ser Lys Leu Ala Ser Gly Val Pro Ser Arg Phe Ser Gly Ser
    50              55              60

Gly Ser Gly Thr Glu Phe Thr Leu Thr Ile Ser Ser Leu Gln Pro Asp
65              70              75              80

Asp Phe Ala Thr Tyr Tyr Cys Phe Gln Gly Ser Gly Tyr Pro Phe Thr
            85              90              95

Phe Gly Gly Gly Thr Lys Leu Glu Ile Lys
            100             105

EP 2 660 324 A1

```
<210>  12
<211>  120
<212>  PRT
<213>  homo sapiens

<400>  12

Gln Val Thr Leu Arg Glu Ser Gly Pro Ala Leu Val Lys Pro Thr Gln
1               5               10              15

Thr Leu Thr Leu Thr Cys Thr Phe Ser Gly Phe Ser Leu Ser Thr Ser
            20              25              30

Gly Met Ser Val Gly Trp Ile Arg Gln Pro Pro Gly Lys Ala Leu Glu
            35              40              45

Trp Leu Ala Asp Ile Trp Trp Asp Asp Lys Lys Asp Tyr Asn Pro Ser
            50              55              60

Leu Lys Ser Arg Leu Thr Ile Ser Lys Asp Thr Ser Lys Asn Gln Val
65              70              75              80

Val Leu Lys Val Thr Asn Met Asp Pro Ala Asp Thr Ala Thr Tyr Tyr
                85              90              95

Cys Ala Arg Ser Met Ile Thr Asn Trp Tyr Phe Asp Val Trp Gly Ala
            100             105             110

Gly Thr Thr Val Thr Val Ser Ser
        115             120


<210>  13
<211>  107
<212>  PRT
<213>  homo sapiens

<400>  13

Asp Ile Gln Met Thr Gln Ser Pro Ser Thr Leu Ser Ala Ser Val Gly
1               5               10              15

Asp Arg Val Thr Ile Thr Cys Ser Ala Ser Ser Arg Val Gly Tyr Met
            20              25              30

His Trp Tyr Gln Gln Lys Pro Gly Lys Ala Pro Lys Leu Leu Ile Tyr
        35              40              45

Asp Thr Ser Lys Leu Ala Ser Gly Val Pro Ser Arg Phe Ser Gly Ser
        50              55              60

Gly Ser Gly Thr Glu Phe Thr Leu Thr Ile Ser Ser Leu Gln Pro Asp
65              70              75              80

Asp Phe Phe Ala Thr Tyr Tyr Cys Phe Gln Gly Ser Gly Tyr Pro Phe
                85              90              95
```

24

Thr Phe Gly Gly Gly Thr Lys Val Glu Ile Lys
                100               105


<210> 14
<211> 120
<212> PRT
<213> homo sapiens

<400> 14

Gln Val Thr Leu Arg Glu Ser Gly Pro Ala Leu Val Lys Pro Thr Gln
1               5               10              15

Thr Leu Thr Leu Thr Cys Thr Phe Ser Gly Phe Ser Leu Ser Thr Ala
           20              25              30

Gly Met Ser Val Gly Trp Ile Arg Gln Pro Pro Gly Lys Ala Leu Glu
           35              40              45

Trp Leu Ala Asp Ile Trp Trp Asp Asp Lys Lys His Tyr Asn Pro Ser
     50              55              60

Leu Lys Asp Arg Leu Thr Ile Ser Lys Asp Thr Ser Lys Asn Gln Val
65              70              75              80

Val Leu Lys Val Thr Asn Met Asp Pro Ala Asp Thr Ala Thr Tyr Tyr
               85              90              95

Cys Ala Arg Asp Met Ile Phe Asn Phe Tyr Phe Asp Val Trp Gly Gln
           100             105             110

Gly Thr Thr Val Thr Val Ser Ser
           115             120


<210> 15
<211> 107
<212> PRT
<213> homo sapiens

<400> 15

Asp Ile Gln Met Thr Gln Ser Pro Ser Ser Leu Ser Ala Ser Val Gly
1               5               10              15

Asp Arg Val Thr Ile Thr Cys Lys Ala Ser Gln Asn Ile Asp Lys Tyr
           20              25              30

Leu Asn Trp Tyr Gln Gln Lys Pro Gly Lys Ala Pro Lys Leu Leu Ile
           35              40              45

Tyr Asn Thr Asn Asn Leu Gln Thr Gly Val Pro Ser Arg Phe Ser Gly
     50              55              60

Ser Gly Ser Gly Thr Asp Phe Thr Phe Thr Ile Ser Ser Leu Gln Pro
65              70              75              80

Glu Asp Ile Ala Thr Tyr Tyr Cys Leu Gln His Ile Ser Arg Pro Arg
                85                  90              95

Thr Phe Gly Gln Gly Thr Lys Val Glu Ile Lys
            100             105

<210> 16
<211> 121
<212> PRT
<213> homo sapiens

<400> 16

Gln Val Gln Leu Gln Glu Ser Gly Pro Gly Leu Val Arg Pro Ser Gln
1               5               10              15

Thr Leu Ser Leu Thr Cys Thr Val Ser Gly Phe Thr Phe Thr Asp Phe
            20              25              30

Tyr Met Asn Trp Val Arg Gln Pro Pro Gly Arg Gly Leu Glu Trp Ile
        35              40              45

Gly Phe Ile Arg Asp Lys Ala Lys Gly Tyr Thr Thr Glu Tyr Asn Pro
    50              55              60

Ser Val Lys Gly Arg Val Thr Met Leu Val Asp Thr Ser Lys Asn Gln
65              70              75              80

Phe Ser Leu Arg Leu Ser Ser Val Thr Ala Ala Asp Thr Ala Val Tyr
            85              90              95

Tyr Cys Ala Arg Glu Gly His Thr Ala Ala Pro Phe Asp Tyr Trp Gly
            100             105             110

Gln Gly Ser Leu Val Thr Val Ser Ser
        115             120

<210> 17
<211> 107
<212> PRT
<213> homo sapiens

<400> 17

Asp Ile Leu Leu Thr Gln Ser Pro Val Ile Leu Ser Val Ser Pro Gly
1               5               10              15

Glu Arg Val Ser Phe Ser Cys Arg Ala Ser Gln Ser Ile Gly Thr Asn
            20              25              30

Ile His Trp Tyr Gln Gln Arg Thr Asn Gly Ser Pro Arg Leu Leu Ile
        35              40              45

Lys Tyr Ala Ser Glu Ser Ile Ser Gly Ile Pro Ser Arg Phe Ser Gly
    50              55              60

26

Ser Gly Ser Gly Thr Asp Phe Thr Leu Ser Ile Asn Ser Val Glu Ser
65                70                    75                    80

Glu Asp Ile Ala Asp Tyr Tyr Cys Gln Gln Asn Asn Asn Trp Pro Thr
                85                    90                    95

Thr Phe Gly Ala Gly Thr Lys Leu Glu Leu Lys
            100                 105

<210>   18
<211>   119
<212>   PRT
<213>   homo sapiens

<400>   18

Gln Val Gln Leu Lys Gln Ser Gly Pro Gly Leu Val Gln Pro Ser Gln
1                 5                 10                    15

Ser Leu Ser Ile Thr Cys Thr Val Ser Gly Phe Ser Leu Thr Asn Tyr
            20                    25                    30

Gly Val His Trp Val Arg Gln Ser Pro Gly Lys Gly Leu Glu Trp Leu
        35                    40                    45

Gly Val Ile Trp Ser Gly Gly Asn Thr Asp Tyr Asn Thr Pro Phe Thr
    50                    55                    60

Ser Arg Leu Ser Ile Asn Lys Asp Asn Ser Lys Ser Gln Val Phe Phe
65                70                    75                    80

Lys Met Asn Ser Leu Gln Ser Asn Asp Thr Ala Ile Tyr Tyr Cys Ala
                85                    90                    95

Arg Ala Leu Thr Tyr Tyr Asp Tyr Glu Phe Ala Tyr Trp Gly Gln Gly
            100                 105                   110

Thr Leu Val Thr Val Ser Ala
            115

<210>   19
<211>   106
<212>   PRT
<213>   homo sapiens

<400>   19

Gln Ile Val Leu Ser Gln Ser Pro Ala Ile Leu Ser Ala Ser Pro Gly
1                 5                 10                    15

Glu Lys Val Thr Met Thr Cys Arg Ala Ser Ser Ser Val Ser Tyr Ile
            20                    25                    30

His Trp Phe Gln Gln Lys Pro Gly Ser Ser Pro Lys Pro Trp Ile Tyr
        35                    40                    45

27

```
Ala Thr Ser Asn Leu Ala Ser Gly Val Pro Val Arg Phe Ser Gly Ser
    50                  55              60

Gly Ser Gly Thr Ser Tyr Ser Leu Thr Ile Ser Arg Val Glu Ala Glu
65              70              75                          80

Asp Ala Ala Thr Tyr Tyr Cys Gln Gln Trp Thr Ser Asn Pro Pro Thr
              85              90              95

Phe Gly Gly Gly Thr Lys Leu Glu Ile Lys
            100             105
```

```
<210>  20
<211>  121
<212>  PRT
<213>  homo sapiens

<400>  20
```

```
Gln Val Gln Leu Gln Gln Pro Gly Ala Glu Leu Val Lys Pro Gly Ala
1               5               10                      15

Ser Val Lys Met Ser Cys Lys Ala Ser Gly Tyr Thr Phe Thr Ser Tyr
            20              25              30

Asn Met His Trp Val Lys Gln Thr Pro Gly Arg Gly Leu Glu Trp Ile
        35              40              45

Gly Ala Ile Tyr Pro Gly Asn Gly Asp Thr Ser Tyr Asn Gln Lys Phe
        50              55              60

Lys Gly Lys Ala Thr Leu Thr Ala Asp Lys Ser Ser Ser Thr Ala Tyr
65              70              75                          80

Met Gln Leu Ser Ser Leu Thr Ser Glu Asp Ser Ala Val Tyr Tyr Cys
              85              90              95

Ala Arg Ser Thr Tyr Tyr Gly Gly Asp Trp Tyr Phe Asn Val Trp Gly
            100             105             110

Ala Gly Thr Thr Val Thr Val Ser Ala
        115             120
```

Description

FIELD OF THE INVENTION

The present invention is directed to methods for producing therapeutic antibodies, functional fragments or derivatives thereof in specific microalgae, said therapeutic antibodies, functional fragment or derivatives thereof being secreted in the liquid culture medium and preferably having an enhanced ADCC and a low fucose content.

BACKGROUND OF THE INVENTION

Over the last 30 years, considerable progress has been made in medical treatment thanks to the development of biotechnology-derived pharmaceuticals. The vast majority of approved products consist of proteins and polypeptides with more than 50% being produced from recombinant mammalian cell-culture expression systems (see Walsh (2010) Biopharmaceutical benchmarks 2010. Nature Biotech., 28: 917-924). This is due to the importance of post-translational modifications (PTMs), particularly glycosylation, on biochemical and therapeutic properties. Amongst biotherapeutics, monoclonal antibodies (mAbs) and mAbs-based products represent the fastest growing segment with sales above $40 billion. They gather top-selling products in areas such as rheumatoid arthritis (e.g. Remicade®, Enbrel®, Humira®) or cancers (e.g. Avastin®, Rituxan®, Herceptin®, Erbitux®).

The vast majority of monoclonal antibodies available on the market or under development are produced in mammalian cells. The workhorse for biomanufacturing is Chinese Hamster Ovary (CHO) cells, a host benefiting from an extensive know-how as well as the possibility to manufacture at large scale in 10,000 L bioreactors. However, mammalian cells suffer from drawbacks and limitations in regard to potential risk of contamination by human pathogens including viruses and high manufacturing cost. Consequently, these challenges have driven the development of novel expression technologies. Amongst them, plant production systems have received a considerable interest due to their low-cost potential. Several biologicals have now been expressed in plants and few of them are already undergoing clinical phases (as reviewed by Paul and Ma (2011) Plant-made pharmaceuticals: Leading products and production platforms. Biotechnol. Appl. Biochem. 58: 58-67). While being attractive, the specificity of the production process, i.e. cultivation, harvesting and primary processing, will make difficult the adoption of such technologies by the pharmaceutical industry. Thus, manufacturing of plant-made pharmaceuticals according to good manufacturing practice (GMP), although not infeasible, requires considerable adaptations. Downstream processing is also more complex as expression of recombinant products is mainly done in whole-plant whereas those produced in mammalian cells are typically recovered from the cell culture medium.

In contrast to plant, microalgae are a very diverse and heterogeneous group of photosynthetic microorganisms that possess very favorable characteristics for application in biomanufacturing. Indeed, microalgae naturally grow as cell suspension and can therefore be cultivated in confined bioreactors or even fermentors for heterotrophic species. High yield in biomass can also be reached in simple and chemically defined media containing only minerals and vitamins. Evidences that industrial processes run under GMP conditions can be operated with microalgal cells exist as illustrated by the production of docosahexaenoic acid (DHA) used for infant nutrition (Spolaore et al. (2006) Commercial applications of microalgae. J. Biosci. Bioeng. 101: 87-96).

The possibility to express monoclonal antibodies or fragments thereof in microalgal cells has been detailed in patent applications (see for example WO2003/091413 and WO2009/064777). These inventions relate to the production of monoclonal antibodies by genetic transformation of the chloroplastic genome of the green microalgae *Chlamydomonas reinhardtii.* However, the main limitation of plastid expression pertains to the prokaryotic-like nature of the chloroplastic genome itself which does not allow the production of glycoproteins (Rasala and Mayfield (2011) The microalga Chlamydomonas reinhardtii as a platform for the production of human protein therapeutics. Bioeng. Bugs 2:50-54). While aglycosylated antibodies and fragments thereof can be effective (e.g. Cimzia® and Lucentis® produced in *E.coli*), most monoclonal antibodies must bear N-glycans on the constant region of the heavy chain to display their full biological activities. Thus, several monoclonal antibodies that target surface epitope on tumor cells used the so-called antibody-dependant cell-mediated cytotoxicity (ADCC) which is influenced by antibodies glycosylation (as reviewed by Beck et al. (2008) Trends in glycosylation, glycoanalysis and glycoengineering of therapeutic antibodies and Fc-fusion proteins. Current Pharm. Biotechnol. 9:482-501).

Recently, Hempel et al. (2011, PLos ONE 6(12):e28424. Doi:10.1371/journal.pone.0028424) have expressed, in the aim of a diagnosis only, a non therapeutic antibody in the microalga *Phaeodactylum tricornutum* which is not secreted but hold in the endoplasmic reticulum of said microalga.

Hempel et al. does not suggest the secretion of said antibody in said microalga but on the contrary it suggests avoiding transit of said antibody through the Golgi apparatus so as to avoid post-translational modifications and consequently impaired folding of the antibody.

Moreover, apart from the association of said non therapeutic antibody with its antigen, the functionality of said antibody resulting from the structure of its constant regions has not been demonstrated.

The inventors have surprisingly discovered that certain microalgae species can be used as a very efficient cell factory for the production and secretion into the extracellular media of monoclonal antibodies. Advantageously, N-glycans of monoclonal antibodies produced by means of the present invention have a very low level of fucose and therefore a higher ADCC beneficial for anti-tumor or anti-infective activities. The present invention offers benefit over existing technological approaches to reduce fucose content in that it does not require complex engineering of the N-glycosylation pathways (see Beck *et al.* (2008) as disclosed previously).

## SUMMARY OF THE INVENTION

A first aspect of the invention concerns a transformed microalga comprising a nucleic acid sequence operatively linked to a promoter, wherein said nucleic acid sequence encodes an amino acid sequence comprising:

(i) an heterologous signal peptide; and
(ii) a therapeutic antibody, a functional fragment or a derivative thereof,

said transformed microalga expressing the therapeutic antibody, functional fragment or derivative thereof secreted in the extracellular media, and
said microalga being selected among green algae except Volvocales, and among red algae, chromalveolates, and euglenids.

In a preferred embodiment, said transformed microalga is selected among the division of Chlorophytes (except Volvocales), Rhodophytes, Dinoflagellates, Diatoms, Eustigmatophytes, Haptophytes and Euglenids, preferably among the genus *Tetraselmis, Porphyridium, Symbiodinium, Thalassiosira, Nannochloropsis, Emiliania, Pavlova, Isochrysis, Eutrepliella, Euglena,* most preferably among the genus *Nannochloropsis, Isochrysis* and *Tetraselmis,* and still most preferably among the species *Nannochloropsis oculata, Isochrysis galbana* and *Tetraselmis suecica.*

In a preferred embodiment, said therapeutic antibody, functional fragment or derivative thereof according to the invention has an increased antibody-dependant cell-mediated cytotoxicity (ADCC), preferably an increase in ADCC of at least about 10%, 20%, 30%, 40%, 50%, 60%, 70%, 80%, 90%, 100%, 150%, 200%, 250%, 300%, 325%, 400%, or 500% in comparison to a control.

In a preferred embodiment, said therapeutic antibody, functional fragment or derivative thereof according to the invention has a low fucose content, preferably said therapeutic antibody, functional fragment or derivative thereof contains less than 20%, preferably less than 15%, 10%, and even more preferably less than 5%, 1% or 0,1% of fucosylated oligosaccharides on its N-glycan structures.

Another aspect of the invention concerns a method for producing a therapeutic antibody, a functional fragment or a derivative thereof which is secreted in the extracellular medium, said method comprising the steps of:

(i) culturing a transformed microalga according to the invention;
(ii) harvesting the extracellular medium of said culture; and
(iii) purifying the therapeutic antibody, a functional fragment or a derivative
thereof, which is secreted in said extracellular medium.

In a preferred embodiment, said method further comprises a step of determining

■ the ADCC of said therapeutic antibody, functional fragment or derivative thereof, and/or
■ the glycosylation pattern of said therapeutic antibody, functional fragment or derivative thereof, and/or
■ the fucose content of said therapeutic antibody, functional fragment or derivative thereof.

Another aspect of the invention concerns the use of a transformed microalga according to the invention for the production and the secretion of a therapeutic antibody, a functional fragment or a derivative thereof in the extracellular medium as disclosed previously.

Another aspect of the invention concerns a therapeutic antibody, a functional fragment or a derivative thereof produced and secreted in the extracellular medium of microalgae by a method as disclosed previously.

In a preferred embodiment, said therapeutic antibody, a functional fragment or a derivative thereof presents an increase antibody-dependant cell-mediated cytotoxicity (ADCC), preferably an increase in ADCC of at least about 10%, 20%, 30%, 40%, 50%, 60%, 70%, 80%, 90%, 100%, 150%, 200%, 250%, 300%, 325%, 400%, or 500% in comparison to a control.

In another preferred embodiment, said therapeutic antibody, a functional fragment or a derivative thereof according to the invention has a low fucose content has a low fucose content, and even more preferably contains less than 20%, preferably less than 15%, 10%, and even more preferably less than 5%, 1% or 0,1% of fucosylated oligosaccharides on its N-glycan structures.

Finally, another aspect of the invention concerns a pharmaceutical composition comprising a therapeutic antibody, a functional fragment or a derivative thereof according to the invention.

**BRIEF DESCRIPTION OF DRAWINGS**

Figure 1: Immunoblotting analysis under non-reducing conditions of cetuximab produced and secreted by *Nannochloropsis oculata.*

Figure 2: Dot blot analysis of cetuximab produced and secreted by *Isochrysis galbana.*

**DETAILED DESCRIPTION OF THE INVENTION**

The invention aims to provide a new system for producing therapeutic antibodies, functional fragments or derivatives thereof in specific microalgae, said therapeutic antibodies, functional fragments or derivatives thereof being secreted in the extracellular medium of said microalgae.

More preferably said invention aims to provide a new system which allows the production of therapeutic antibodies, functional fragments or derivatives thereof, with increased antibody-dependant cell-mediated cytotoxicity (ADCC) and a low level of fucose in the liquid culture medium of transformed microalgae.

Therefore, a first object of the invention is a transformed microalga comprising a nucleic acid sequence operatively linked to a promoter, wherein said nucleic acid sequence encodes an amino acid sequence comprising:

(i) an heterologous signal peptide; and
(ii) a therapeutic antibody, a functional fragment or a derivative thereof,

said transformed microalga expressing the therapeutic antibody, functional fragment or derivative thereof secreted in the extracellular media, and said microalga being selected among green algae except Volvocales, and among red algae, chromalveolates, and euglenids.

In a preferred embodiment, said microalga according to the invention is selected among the division of Chlorophytes (except Volvocales), Rhodophytes, Dinoflagellates, Diatoms, Eustigmatophytes, Haptophytes and Euglenids.

In another preferred embodiment, said microalga according to the invention is selected among the genus *Tetraselmis, Porphyridium, Symbiodinium, Thalassiosira, Nannochloropsis, Emiliania, Pavlova, Isochrysis, Eutreptiella, Euglena.*

In another still preferred embodiment, said microalga according to the invention is selected among the genus *Nannochloropsis, Isochrysis* and *Tetraselmis.*

In another still preferred embodiment, said microalga according to the invention is selected among the species *Nannochloropsis oculata, Isochrysis galbana* and *Tetraselmis suecica.*

Said microalga secretes glycoproteins, which glycoproteins show a low fucose content on its N-glycans. Optionally, said microalga has optionally a heterotrophic growth.

Transformation of microalgae can be carried out by conventional methods such as microparticles bombardment, electroporation, glass beads, polyethylene glycol (PEG). Such a protocol is disclosed in the examples.

In an embodiment of the invention, nucleotide sequences may be introduced into microalgae of the present invention via a plasmid, virus sequences, double or simple strand DNA, circular or linear DNA.

In another embodiment of the invention, it is generally desirable to include into each nucleotide sequences or vectors at least one selectable marker to allow selection of microalgae that have been stably transformed. Examples of such markers are antibiotic resistant genes such as *sh ble* gene enabling resistance to zeocin, *nat* or *sat-1* genes enabling resistance to nourseothricin, *aph8* enabling resistance to paromomycin, *nptII* enabling resistance to G418.

After transformation of microalgae of the present invention, transformants producing the desired therapeutic antibodies, functional fragments or derivatives thereof secreted in the culture media are selected. Selection can be carried out by one or more conventional methods comprising: enzyme-linked immunosorbent assay (ELISA), mass spectroscopy such as MALDI-TOF-MS, ESI-MS chromatography, spectrophotometer, fluorimeter, immunocytochemistry by exposing cells to an antibody having a specific affinity for the desired therapeutic antibodies, functional fragments or derivatives thereof.

The term "nucleic acid sequence" used herein refers to DNA sequences (e.g., cDNA or genomic or synthetic DNA) and RNA sequences (e. g., mRNA or synthetic RNA), as well as analogs of DNA or RNA containing non-natural nucleotide analogs, non-native internucleoside bonds, or both. Preferably, said nucleic acid sequence is a DNA sequence. The nucleic acid can be in any topological conformation, like linear or circular.

"Operatively linked" promoter refers to a linkage in which the promoter is contiguous with the gene of interest to control the expression of said gene.

Examples of promoter that drives expression of a polypeptide in transformed microalgae include, but are not restricted to, nuclear promoters such as fcpA and fcpB from *Phaeodactylum tricornutum* (Zavlaskaïa and Lippmeier (2000, Transformation of the diatom Phaeodactylum tricornutum (Bacillariophyccac) with a variety of selectable marker and reporter genes. J. Phycol. 36, 379-386)), VCP1 and VCP2 endogenous promoters from Nannochloropsis sp. (Kilian et al. (2011, High-efficiency homologous recombination in the oil-producing alga Nannochloropsis sp. Proc. Natl. Acad. Sci.USA, 108:21265-21269), heterologous promoters as CaMV35S (pCambia2300 AF234315.1).

The nucleic acid sequence used for the transformation of microalgae of the present invention encodes an amino acid sequence comprising a heterologous signal peptide, said heterologous signal peptide enabling the secretion of a therapeutic antibody, a functional fragment or a derivative thereof.

The term "peptide" as used herein refers to an amino acid sequence that is typically less than 50 amino acids long and more typically less than 30 amino acids long.

The term "signal peptide" as used herein refers to an amino acid sequence which is generally located at the amino terminal end of the amino acid sequence of a therapeutic antibody or functional fragment thereof. The signal peptide mediates the translocation of said therapeutic antibody, functional fragment or derivative thereof through the secretion pathway and leads to the secretion of said therapeutic antibody, functional fragment or derivative thereof in the extracellular medium.

As used herein, the term "secretion pathway" refers to the process used by a cell to secrete proteins out of the intracellular compartment. Such pathway comprises a step of translocation of a polypeptide across the endoplasmic reticulum membrane, followed by the transport of the polypeptide in the Golgi apparatus, said polypeptide being subsequently released in the extracellular medium of the cell by secretory vesicles. Post-translational modifications necessary to obtain mature proteins, such as glycosylation or disulfide bonds formation, are operated on proteins during said secretion pathway.

Preferably, the signal peptide leading to the secretion of a therapeutic antibody, functional fragment or derivative thereof according to the invention in the extracellular medium of transformed microalgae is located at its amino-terminal end.

This signal peptide is typically 15-30 amino acids long, and presents a 3 domains structure (von Heijne (1990) The signal Peptide, J. Membr. Biol., 115: 195-201; Emanuelsson et al. (2007) Locating proteins in the cell using TargetP, SignalP and related tools. Nat. Protoc. 2: 953-971), which are as follows:

(i) an N-terminal region (n-region) containing positively charged amino acids, such as Arginine (R), Histidine (H) or Lysine (K);
(ii) a central hydrophobic region (h-region) of at least 6 amino acids containing hydrophobic amino acids such as Alanine (A), Cysteine (C), Glycine (G), Isoleucine (1), Leucine (L), Methionine (M), Phenylalanine (F), Proline (P), Tryptophan (W) or Valine (V); and
(iii) a C-terminal region (c-region) of polar uncharged amino acids such as Asparagine (R), Glutamine (Q), Serine (S), Threonine (T) or Tyrosine (Y). Said C-region often contains a helix-breaking proline or glycine that helps define a cleavage site. Small uncharged residues in positions -3 and -1 (defined as the number of residue before the cleavage site) are usually requires for an efficient cleavage by signal peptidase following the translocation across the endoplasmic reticulum membrane (von Heijne (1990) as disclosed previously; Vernet and Schatz (1988) Protein translocation across membranes, Science, 241: 1307-1313).

A person skilled in the art is able to simply identify a signal peptide in an amino acid sequence, for example by using the SignalP 4.0 Server (accessible on line at http://www.cbs.dtu.dk/services/SignalP/) which predicts the presence and location of signal peptide cleavage sites in amino acid sequences from different organisms by using two different models: the Neural networks and the Hidden Markov models (Petersen et al. (2011) SignalP 4.0: discriminating signal peptides from transmembrane regions, Nat. Methods, 8: 785-786).

The term "heterologous", with reference to a signal peptide according to the invention, means an amino acid sequence which does not exist in the corresponding microalga before its transformation. It is intended that the term encompasses proteins that are encoded by wild-type genes, mutated genes, and/or synthetic genes.

An antibody is an immunoglobulin molecule corresponding to a tetramer comprising four polypeptide chains, two identical heavy (H) chains (about 50-70 kDa when full length) and two identical light (L) chains (about 25 kDa when full length) inter-connected by disulfide bonds. Light chains are classified as kappa and lambda. Heavy chains are classified as gamma, mu, alpha, delta, or epsilon, and define the antibody's isotype as IgG, IgM, IgA, IgD, and IgE, respectively. Each heavy chain is comprised of a N-term heavy chain variable region (abbreviated herein as HCVR) and a heavy chain constant region. The heavy chain constant region is comprised of three domains (CH1, CH2, and CH3) for IgG, IgD, and IgA; and 4 domains (CH1, CH2, CH3, andCH4) for IgM and IgE. Each light chain is comprised of a N-term light chain variable region (abbreviated herein as LCVR) and a light chain constant region. The light chain constant region is comprised of one domain, CL. The HCVR and LCVR regions can be further subdivided into regions of hypervariability, termed complementarity determining regions (CDRs), interspersed with regions that are more conserved, termed framework regions (FR). Each HCVR and LCVR is composed of three CDRs and four FRs, arranged from amino-terminus to carboxy-terminus in the following order: FR1, CDR1, FR2, CDR2, FR3, CDR3, FR4. The assignment of amino acids to each domain is in accordance with well-known conventions (KABAT, "Sequences of Proteins of Immunological Interest", National Institutes of Health, Bethesda, Md., 1987 and 1991; Chothia and Lesk (1987) Canonical structures for the hypervariable regions of immunoglobulins, J. Mol. Biol., 196: 901-17; Chothia et al. (1989) Conformations of immunoglobulin hypervariable regions, Nature, 342: 878-83). The functional ability of the antibody to bind a particular antigen depends on the variable regions of each light/heavy chain pair, and is largely determined by the CDRs.

The term "antibody", as used herein, refers to a monoclonal antibody *per se.* A monoclonal antibody can be a human antibody, chimeric antibody and/or humanized antibody.

The term "therapeutic" referring to an antibody, a functional fragment or derivative thereof designates more specifically any antibody, functional fragment or derivative thereof that functions to deplete target cells in a patient. Specific examples of such target cells include tumor cells, virus -infected cells, allogenic cells, pathological immunocompetent cells (e.g. , B lymphocytes, T lymphocytes, antigen-presenting cells, etc.) involved in cancers, allergies, autoimmune diseases, allogenic reactions. Most preferred target cells within the context of this invention arc tumor cells and virus -infected cells. The therapeutic antibodies may, for instance, mediate a cytotoxic effect or cell lysis, particularly by antibody-dependant cell-mediated cytotoxicity (ADCC). Therapeutic antibodies according to the invention may be directed to epitopes of surface which are overexpressed by cancer cells, or directed to viral epitopes of surface.

In a preferred embodiment, a therapeutic antibody according to the invention is a monoclonal antibody.

In another preferred embodiment, a therapeutic antibody is a human antibody.

In another preferred embodiment, a therapeutic antibody is a chimeric antibody.

By "chimeric antibody" is meant an antibody that is composed of variables regions from a murine immunoglobulin and of constant regions of a human immunoglobulin. This alteration consists simply of substituting the constant region of a murine antibody by the human constant region, thus resulting in a human/murine chimera which may have sufficiently low immunogenicity to be acceptable for pharmaceutical use.

A number of methods for producing such chimeric antibodies have been reported, thus forming part of the general knowledge of the skilled artisan (See, e.g., U.S. Pat. No. 5,225,539).

In a preferred embodiment, said antibody is a chimeric antibody and the light and heavy chain framework sequences are from mouse immunoglobulin light and heavy chains respectively.

Preferably, said chimeric antibody further comprises the constant regions from human light and heavy chains.

In another preferred embodiment, a therapeutic antibody is a humanized antibody.

By "humanized antibody" is meant an antibody that is composed partially or fully of amino acid sequences derived from a human antibody by altering the sequence of an antibody having non-human complementarity determining regions (CDR). This humanization of the variable region of the antibody and eventually the CDR is made by techniques that are by now well known in the art.

As an example, British Patent Application GB 2188638A and US Patent No. 5,585,089 disclose processes wherein recombinant antibodies are produced where the only portion of the antibody that is substituted is the complementarity determining region, or "CDR". The CDR grafting technique has been used to generate antibodies which consist of murine CDRs, and human variable region framework and constant regions (See. e. g., Riechmann et al. (1988) Reshaping human antibodies for therapy, Nature, 332: 323-327). These antibodies retain the human constant regions that are necessary for Fc dependent effector function, but are much less likely to evoke an immune response against the antibody.

Preferably, a humanized antibody again refers to an antibody comprising a human framework, at least one CDR from a non-human antibody, and in which any constant region present is substantially identical to a human immunoglobulin constant region, i.e., at least about 85 or 90%, preferably at least 95% identical. Hence, all parts of a humanized antibody, except possibly the CDRs, are substantially identical to corresponding parts of one or more native human immunoglobulin sequences. For example, a humanized immunoglobulin would typically not encompass a chimeric mouse variable region/human constant region antibody.

In another preferred embodiment, said antibody is a humanized antibody and the light and heavy chain framework sequences are from humanized immunoglobulin light and heavy chains respectively.

Preferably, said humanized antibody further comprises the constant regions from human light and heavy chains.

Most preferably, the constant regions from human light and heavy chains are selected in a group comprising light and heavy chain constant regions corresponding to IgG1.

Examples of constant regions from human light and heavy chains are well known in the art. An example of human gamma 1 constant region is described in Shitara et al (1993, Chimeric antiganglioside GM2 antibody with antitumor activity, Cancer Immunol. Immunother., 36: 373-380).

Other sequences are possible for the light and heavy chains for the humanized antibodies of the present invention. The immunoglobulins can have two pairs of light chain/heavy chain complexes, at least one chain comprising one or more mouse complementarity determining regions functionally joined to human framework region segments.

In another preferred embodiment, a therapeutic antibody is selected among the group comprising rituximab, trastuzumab, cetuximab, motavizumab, palivizumab, alemtuzumab, but also comprising for instance, benralizumab, catumaxomab, daratumumab, elotuzumab, epratuzumab, farletuzumab, galiximab, gemtuzumab ozogamicin, ibritumomab tiuxetan, lumiliximab, necitumumab, nimotuzumab, ocrelizumab, ofatumumab, oregovomab, pertuzumab, tositumomab, zalutumumab, and zanolimumab, preferably the cetuximab.

Advantageously, preferred light chain variable region (LCVR) and heavy chain variable region (HCVR) are selected in the group comprising but not limited to chains as depicted in Table 1.

Table 1. Preferred embodiment regarding an antibody according to the invention

| Monoclonal antibody | Epitope | Variable light chain (LCVR) | Variable heavy chain (HCVR) |
|---|---|---|---|
| Trastuzumab | HER2 | SEQ ID N°9 | SEQ ID N°10 |
| Palivizumab | F protein of respiratory Syncytial virus | SEQ ID N°11 | SEQ ID N°12 |
| Motavizumab | F protein of respiratory Syncytial virus | SEQ ID N°13 | SEQ ID N°14 |
| Alemtuzumab | CD52 | SEQ ID N°15 | SEQ ID N°16 |
| Celuximab | EGFR | SEQ ID N°17 | SEQ ID N°18 |
| Rituximab | CD20 | SEQ ID N°19 | SEQ ID N°20 |

Advantageously said antibody comprises the light chain variable region (LCVR) having an amino acid sequence selected in the group comprising but not limited to SEQ ID NO:9, SEQ NO:11, SEQ ID NO:13, SEQ ID NO:15, SEQ ID NO:17, and SEQ ID NO:19.

Most preferably, said light chain variable region (LCVR) comprises the amino acid sequence comprising SEQ ID NO:17.

Again advantageously, said antibody comprises the heavy chain variable region (HCVR) with an amino acid sequence selected in the group comprising but not limited to SEQ ID NO:10, SEQ NO:12, SEQ ID NO:14, SEQ ID NO:16, SEQ ID NO:18 and SEQ ID NO:20.

Most preferably, said heavy chain variable region (HCVR) comprises an amino acid sequence comprising SEQ ID NO:18.

In another preferred embodiment, the light chain of a therapeutic antibody of the invention corresponds to a sequence selected in the group comprising SEQ ID NO:1 and SEQ ID NO:7, preferably SEQ ID NO:1.

In another still preferred embodiment, the heavy chain of a therapeutic antibody of the invention corresponds to a sequence selected in the group comprising SEQ ID NO:2 and SEQ ID NO:8, preferably SEQ ID NO:2.

Such antibodies may be used according to clinical protocols that have been authorized for use in human subjects. One skilled in the art would recognize that other therapeutic antibodies are useful in the methods of the invention.

The term "functional fragments" as used herein refers to antibody fragment capable of reacting with its reaction target, such as for example, but not limited to, antigens comprising surface or tumoral antigens, receptors, etc. Such fragments can be simply identified by the skilled person and comprise, as an example, $F_{ab}$ fragment (e.g., by papain digestion), $F_{ab}$' fragment (e.g., by pepsin digestion and partial reduction), $F(_{ab}')_2$ fragment (e.g., by pepsin digestion), $F_{acb}$ (e.g., by plasmin digestion), $F_d$ (e.g., by pepsin digestion, partial reduction and reaggregation), and also $scF_v$ (single chain Fv; e.g., by molecular biology techniques) fragment are encompassed by the invention.

Such fragments can be produced by enzymatic cleavage, synthetic or recombinant techniques, as known in the art and/or as described herein. Antibodies can also be produced in a variety of truncated forms using antibody genes in which one or more stop codons have been introduced upstream of the natural stop site. For example, a combination gene encoding a $F(_{ab}')_2$ heavy chain portion can be designed to include DNA sequences encoding the $CH_1$ domain and/or hinge region of the heavy chain. The various portions of antibodies can be joined together chemically by conventional techniques, or can be prepared as a contiguous protein using genetic engineering techniques.

As used herein, the term "derivative" refers to a polypeptide having a percentage of identity of at least 90% with the complete amino acid sequence of a therapeutic antibody or functional fragment thereof as disclosed previously and having the same activity.

Preferably, a derivative has a percentage of identity of at least 95% with said amino acid sequence, and preferably of at least 99% with said amino acid sequence.

As used herein, "percentage of identity" between two amino acids sequences, means the percentage of identical amino-acids, between the two sequences to he compared, obtained with the best alignment of said sequences, this percentage being purely statistical and the differences between these two sequences being randomly spread over the amino acids sequences. As used herein, "best alignment" or "optimal alignment", means the alignment for which the determined percentage of identity (see below) is the highest. Sequences comparison between two amino acids sequences are usually realized by comparing these sequences that have been previously aligned according to the best alignment; this comparison is realized on segments of comparison in order to identify and compare the local regions of similarity. The best sequences alignment to perform comparison can be realized by using computer softwares using algorithms such as GAP, BESTFIT, BLAST P, BLAST N, FASTA, TFASTA in the Wisconsin Genetics software Package, Genetics Computer Group, 575 Science Dr., Madison, WI USA. To get the best local alignment, one can preferably used BLAST software, with the BLOSUM 62 matrix, preferably the PAM 30 matrix. The identity percentage between two sequences of amino acids is determined by comparing these two sequences optimally aligned, the amino acids sequences being able to comprise additions or deletions in respect to the reference sequence in order to get the optimal alignment between these two sequences. The percentage of identity is calculated by determining the number of identical position between these two sequences, and dividing this number by the total number of compared positions, and by multiplying the result obtained by 100 to get the percentage of identity between these two sequences.

Surprisingly the inventors of the present invention have found in their experiments that antibodies, functional fragments or derivatives thereof that were produced and secreted in the extracellular medium of transformed microalgae of the present invention could also induce an enhanced ADCC activity, compared to antibodies expressed in mammalian cells.

The system according to the invention thus provides an economical, simple and reliable method for the production and secretion in the liquid culture medium of monoclonal antibodies, or fragments or derivatives thereof, which have a drastically increased ADCC and thus a highly enhanced therapeutic potential.

Therefore, in another preferred embodiment, said therapeutic antibody, functional fragment or derivative thereof produced according to the invention has an enhanced antibody-dependant cell-mediated cytotoxicity (ADCC).

ADCC is a mechanism of cell-mediated immunity whereby an effector cell of the immune system actively lyses a target cell that has been bound by specific antibodies. It is one of the mechanisms through which antibodies, as part of the humoral immune response, can act to limit and contain infection. Classical ADCC-mediating effector cells are natural killer (NK) cells; but monocytes and eosinophils can also mediate ADCC. ADCC is part of the adaptive immune response due to its dependence on a prior antibody response.

The most studied mechanism of action of monoclonal antibodies causing target cell death is ADCC, which is mediated by natural killer (NK) cells. This involves binding of the Fab portion of an antibody to a specific epitope on a cancer cell and subsequent binding of the Fc portion of the antibody to the Fc receptor on the NK cells. This triggers release of perforin, and granzyme that leads to DNA degradation, induces apoptosis and results in cell death. Among the different receptors for the Fc portion of MAbs, the FcgRIIIa, also known as CD16a, plays a major role in ADCC.

"ADCC activity" as used herein refers to an activity to damage a target cell (e.g., tumor cell) by activating an effector cell via the binding of the Fc region of an antibody to an Fc receptor existing on the surface of an effector cell such as a killer cell, a natural killer cell, an activated macrophage or the like. An activity of antibodies, functional fragments or derivatives thereof of the present invention includes ADCC activity. ADCC activity measurements and antitumor experiments can be carried out in accordance using any assay known in the art and commercially available.

The term "enhanced antibody-dependent cellular cyotoxicity", "enhanced ADCC" (e.g. referring to cells), or "increased ADCC" is intended to include any measurable increase in cell lysis when contacted with a therapeutic antibody, functional fragment or derivative thereof according to the invention as compared to the cell killing of the same cell in contact with an antibody produced by conventional Antibody expression systems, e.g., mammalian cells or *E. coli.*

In a preferred embodiment, an increase in ADCC according to the invention may be by at least about 10%, 20%, 30%, 40%, 50%, 60%, 70%, 80%, 90%, 100%, 150%, 200%, 250%, 300%, 325%, 400%, or 500% in comparison to a control.

As used herein, the term "control" refers to the same antibody produced in Chinese Hamster Ovary cells which have not been modified or to the same antibody in its commercial form when it exists.

In another preferred embodiment, said therapeutic antibody or functional fragment thereof produced in said microalga contains a low fucose content.

Proteins expressed in eukaryotic expression systems undergo a process of post-translational modification, which involves glycosylation. Eukaryotic expression systems which have been established today for the production of monoclonal antibodies comprising an Fc region add N-glycans to the polypeptide chains. In all these cases, the N-glycan comprises some fucose residues which are bound either $\alpha$-3-glycosidically or $\alpha$-6-glycosidically to the N-acetyl-glucosamine residue bound to the Asn 297 residue (EU numbering) of the polypeptide chain.

In contrast thereto, microalgae of the present invention produce N-glycan structures on the Asn 297 residue (EU numbering) which are significantly different from the glycoslation patterns produced by the above mentioned expression systems in that it has a low fucose content.

As used herein, the term "fucose content" or "fucose level" refers to the amount of fucose present on the N-glycans structures of said therapeutic antibody, functional fragment or derivative thereof. The "fucose content" represents the amount of fucosylated oligosaccharides as a percentage of the total oligosaccharides on said therapeutic antibody, functional fragment or derivative thereof produced in microalgae of the present invention.

As used herein, the term "fucosylated oligosaccharides » refers to N-glycans attached to the Asn 297 residue and comprising a fucose localized on $\alpha$ (1-3) or $\alpha$ (1-6) positions.

As used herein, total oligosaccharides refers to the total N-glycans that are delivered by the action of deglycosylation enzymes, according to a method known in the art and as disclosed below.

As used herein, the term "low fucose content" means that said therapeutic antibody, functional fragment or derivative thereof produced according to the invention does not comprise more than 10%, preferably 9%, 8%, 7%, 6% and even more preferably 5%, 1% or 0,1% of fucosylated oligosaccharides on N-glycans.

In a preferred embodiment, said therapeutic antibody or functional fragment thereof produced according to the invention contains less than 10%, preferably less than 9%, 8%, 7%, 6% and even more preferably less than 5%, 1% or 0,1% of fucosylated oligosaccharides on its N-glycan structures.

The fucose content of the therapeutic antibody, functional fragment or derivative thereof can be measured by well-known technique from the art such as mass spectrometry analysis. Such a protocol is disclosed in the examples.

In another embodiment of the invention, microalgae used herein for the secretion of polypeptides in the extracellular medium further express an N-acetylglucosaminyltransferase (GnT I, GnT II, GnT III, GnT IV, GnT V or GnT VI), a mannosidase II and galactosyltransferase (GalT) or sialyltransferases (ST), to secrete—glycosylated polypeptides. Glycosylation is dependent on the endogenous machinery present in the host cell chosen for producing and secreting glycosylated polypeptides. Microalgae of the present invention are capable of producing such glycosylated polypeptides in high yield via their endogenous N-glycosylation machinery.

Another object of the invention is a method for producing a therapeutic antibody, a functional fragment or a derivative thereof which is secreted in the extracellular medium, said method comprising the steps of:

(i) culturing a transformed microalga of the present invention as described here above;
(ii) harvesting the extracellular medium of said culture: and
(iii) purifying the therapeutic antibody, a functional fragment or a derivative thereof, which is secreted in said extracellular medium.

In another embodiment of the invention, the method of producing a therapeutic antibody, a functional fragment or a derivative thereof which is secreted in the extracellular medium of transformed microalga comprises a former step of transforming said microalga with a nucleic acid sequence operatively linked to a promoter, wherein said nucleic acid sequence encodes an amino acid sequence comprising an heterologous signal peptide and a therapeutic antibody, a functional fragment or a derivative thereof, said transformed microalga expressing the therapeutic antibody, functional fragment or derivative thereof secreted in the extracellular media.

In another embodiment of the invention, the method of producing secreted therapeutic antibody, functional fragment or derivative thereof in the extracellular medium of transformed microalga further comprises a step of determining the ADCC of said therapeutic antibody, functional fragment or derivative thereof.

Methods for determining the ADCC are well known from the art and some are disclosed previously.

In another embodiment of the invention, the method of producing secreted therapeutic antibody, functional fragment or derivative thereof in the extracellular medium of transformed microalgae of the present invention further comprises a step of determining the glycosylation pattern of said therapeutic antibody, functional fragment or derivative thereof.

This glycosylation pattern can be determined by method well known from the skilled person.

Preliminary information about N-glycosylation of the recombinant polypeptide secreted in the extracellular medium can be obtained by affino- and immunoblotting analysis using specific probes such as lectins (CON A; ECA; SNA; MAA...) and specific N-glycans antibodies (anti-1,2-xylose; anti--1,3-fucose; anti-Neu5Ge, anti-Lewis ...). To investigate the detailed N-glycan profile of recombinant polypeptide, N-linked oligosaccharides is released from the polypeptide in a non specific manner using enzymatic digestion or chemical treatment. The resulting mixture of reducing oligosaccharides can be profiled by HPLC and/or mass spectrometry approaches (ESI-MS-MS and MALDI-TOF essentially). These strategies, coupled to exoglycosidase digestion, enable N-glycan identification and quantification (Séveno et al. (2008, Plant N-glycan profiling of minute amounts of material, Anal. Biochem., 379: 66-72); Stadlmann et al. (2008, Analysis of immunoglobulin glycosylation by LC-ESI-MS of glycopeptides and oligosaccharides, Proteomics, 8: 2858-2871)).

Another alternative to study N-glycosylation profile of recombinant protein is to work directly on its glycopeptides after protease digestion of the protein, purification and mass spectrometry analysis of the glycopeptides as disclosed in Bardor et al. (Monoclonal C5-1 antibody produced in transgenic alfalfa plants exhibits a N-glycosylation that is homogenous and suitable for glyco-engineering into human-compatible structures, Plant Biotechnol. J., 1: 451-462, 2003).

In another embodiment of the invention, the method of producing secreted therapeutic antibody, functional fragment or derivative thereof in the extracellular medium of transformed microalga further comprises a step of determining the fucose content of said therapeutic antibody, functional fragment or derivative thereof.

Methods for determining the fucose content of a protein are well known in the art and examples of such methods are disclosed previously.

In a preferred embodiment, the method of producing a therapeutic antibody, a functional fragment or a derivative thereof secreted in the extracellular medium of transformed microalgae of the present invention leads to the secretion of at least 25%, 30%, 35%, 40%, 45%, 50%, 55%, 60%, 65%, 70%, 75%, 80%, 85%, 90% or 90% of the polypeptide expressed in said microalgae.

Secretion efficiency can be assessed using pulse-chase experiments with radiolabeled amino acids, as described by Jensen et al. (2000, Cell-associated degradation affects the yield of secreted engineered and heterologous proteins in the Bacillus subtilis expression system. Microbiology, 146:2583-2594), except that media are replaced by those used to grow the transformed microalga. The protein to study is then immunoprecipitated on both intracellular and extracellular fractions and subjected to SDS-PAGE electrophoresis and quantified using the phosphor-imaging technology.

The percentage of secretion for any given time can be calculated as follow:

$$\text{QSecreted} + \text{Qinternal} = 100\% \text{ of expressed therapeutic antibodies,}$$

$$\text{functional fragments or derivatives thereof}$$

$$\% \text{ secreted} = (\text{Qsecreted} \times 100\%) / (\text{Qsecreted} + \text{Qinternal})$$

Said formula can be merely explained as followed :

- quantity of the therapeutic antibodies, functional fragments or derivatives thereof in the extracellular medium of transformed microalga (Qsecreted);
- quantity of said therapeutic antibodies, functional fragments or derivatives thereof within the cells of transformed microalga (Qinternal);
- Additioning both quantities as determined previously to obtain the total quantity of produced therapeutic antibodies, functional fragments or derivatives thereof by the transformed microalga, such quantity being equivalent to 100% (100% of expressed polypeptides)
- Multiplying the amount of secreted therapeutic antibodies, functional fragments or derivatives thereof (Qsecreted) by 100%, and dividing the result by the total of therapeutic antibodies, functional fragments or derivatives thereof expressed by the transformed microalga (Qsecreted+Qinternal) to obtain the percentage of therapeutic antibodies, functional fragments or derivatives thereof secreted in the extracellular medium of said microalga (%secreted).

Another object of the invention aims to provide the use of a transformed microalga as previously described for the production and the secretion of a therapeutic antibody, a functional fragment or a derivative thereof as disclosed previously in the extracellular medium.

Another object of the invention concerns a therapeutic antibody, a functional fragment or a derivative thereof produced and secreted in the extracellular medium of microalgae according to the invention.

In a preferred embodiment, said therapeutic antibody, functional fragment or derivative thereof, presents an increased antibody-dependant cell-mediated cytotoxicity (ADCC).

In another preferred embodiment, said therapeutic antibody, functional fragment or derivative thereof according to the invention has a low fucose content, and even more preferably contains less than 10%, preferably less than 9%, 8%, 7%, 6% and even more preferably less than 5%, 13% or 0,1% of fucosylated oligosaccharides on its N-glycan structures.

Another object of the invention concerns a pharmaceutical composition comprising a therapeutic antibody, a functional fragment or a derivative thereof produced by the method as described here above.

Said composition may be in any pharmaceutical form suitable for administration to a patient, including but not limited to solutions, suspensions, lyophilized powders, capsule and tablets.

In a preferred embodiment, said pharmaceutical composition may further comprise a pharmaceutically acceptable carrier selected among pharmaceutically acceptable diluent, excipient or auxiliary.

The pharmaceutical composition of the invention may be formulated for injection, e.g. local injection, transmucosal administration, inhalation, oral administration and more generally any formulation that the skilled person finds appropriate to achieve the desired prognosis and/or diagnosis and/or therapy.

The therapeutic antibody, functional fragment or derivative thereof according to the invention is contained in said pharmaceutical composition in an amount effective to achieve the intended purpose, and in dosages suitable for the chosen route of administration.

More specifically, a therapeutically effective dose means an amount of a compound effective to prevent, alleviate or ameliorate symptoms of the disease or condition of the subject being treated, or to arrest said disease or condition.

Depending on the intended application, the therapeutic antibody, functional fragment or derivative thereof according to the invention may further comprise additional constituents.

In the following, the invention is described in more detail with reference to methods. Yet, no limitation of the invention is intended by the details of the examples. Rather, the invention pertains to any embodiment which comprises details which are not explicitly mentioned in the examples herein, but which the skilled person finds without undue effort.

**EXAMPLES**

**Example 1 Secretion of the chimeric monoclonal antibody cetuximab in the culture medium of transformed Nannochloropsis oculata**

To test the ability of *Nannochloropsis oculata* (*N. Oculata)* to express a fully assembled monoclonal antibody that can be secreted into the extracellular medium, the co-transfection of the nuclear genome was carried out with 2 vectors, each containing either the light chain (SEQ ID N°1) or heavy chain (SEQ ID N°2) of cetuximab.

The light chain sequence encoded for a 230 amino acids precursor containing a 17 amino acids heterologous signal peptide and a 213 amino acids mature protein. The heavy chain sequence encoded for a 469 amino acids precursor containing a 17 amino acids heterologous signal peptide and a 452 amino acids mature protein.

a) Standard culture conditions of *Nannochloropsis oculata*

The microalga *Nannochloropsis oculata* was grown at 20°C under continuous illumination (280-350 $\mu$mol photons.m$^{-2}$.s$^{-1}$), in natural coastal seawater sterilized by 0.22 $\mu$m filtration. This seawater is enriched with nutritive Conway media. For large volume (from 2 litters to 300 liters), cultures were aerated with a 2% $CO_2$/air mixture to maintain the pH in a range of 7.5-8.1. Concentration of culture was estimated on Mallasscz counting cells after fixation of microalgae with a Lugol's solution.

b) Expression constructs for cetuximab

The coding sequences for light and heavy chains were cloned in the pCambia2300 vector (AF234315.1). The gene sh ble conferring the zeocin resistance was cloned between CaMV35S promoter and terminator regulating sequences by XhoI restriction site. The light and heavy chains of cetuximab sequences were synthesized in the expression cassette with CaMV35S promoter and terminator regulating sequences. The restriction sites SacI and HindIII respectively included in 5' and 3' of the cassette, were used for cloning the interest genes in pCambia vector containing the sh ble gene.

As a control, an empty vector lacking cetuximab coding sequences was used.

c) Genetic transformation

The co-transformation was carried out by electroporation following the method described by Kilian et at. (2011, High-efficiency homologous recombination in the oil-producing alga Nannochloropsis sp., 108: 21265-21269).

Cultures of *Nannochloropsis oculata* were harvested at mid-log phase and washed four times in 384 mM D-sorbitol. Cell concentration was adjusted to $1.10^{10}$ cells.mL$^{-1}$ in 384 mM D-sorbitol, and 100 $\mu$L cells and 0.1-1 $\mu$g DNA (equal mix of plasmids containing the light and heavy chains or the empty vector) were used for each electroporation.

Electroporation was performed using the following parameters:

- 2-mm cuvettes
- Exponential decay
- 2200 V field strength

- 50 $\mu$F capacitance and 500 Ohm shunt resistance

After electroporation, cells were immediately transferred to 10 mL fresh culture medium and incubated in low light overnight. Cells (5.10[8]) were plated the next day on Conway agar plates containing 100$\mu$g.ml-1 zeocin. After 2-3 weeks of incubation of the plates, individual clones were picked from the plates and inoculated into liquid medium containing zeocin (100$\mu$g.ml-1).

d) Microalgae DNA extraction

Cells (5.10[8]) transformed by the various vectors were pelleted by centrifugation (2150 g, 15 minutes, 4°C). Microalgal cells were incubated overnight at 4°C with 4 mL of TE NaCl IX buffer (Tris-HCL 0.1 M, EDTA 0.05 M, NaCl 0.1 M, pH 8). 1% SDS, 1% Sarkosyl and 40°C for 90 minutes. A first phenol-chloroform-isoamyl alcohol extraction was carried out to extract an aqueous phase comprising the nucleic acids. RNA contained in the sample was eliminated by an hour incubation at 60°C in the presence of RNase (1 $\mu$g.mL-1). A second phenol-chloroform extraction was carried out, followed by a precipitation with ethanol. The pellet obtained was air-dried and solubilised into 200 $\mu$L of ultrapure sterile water. Quantification of DNA was carried out by spectrophotometry (260 nm) and analysed by agarose gel electrophoresis.

e) Polymerase chain reaction (PCR) analysis

The incorporation of the heterologous chimeric light chain and heavy chain sequences in the genome of *Nannochloropsis oculata* was assessed by PCR analysis. The sequences of primers used for the PCR amplification were 5'-TACCAACAGCGAACGAACG-3' (SEQ ID N°3) and 5'-GTCGACCTTCCATTGGACC-3' (SEQ ID N°4) located in the light chain sequence. For the detection of the heavy chain, the sequences of primers used for the PCR amplification were 5'-CAAGGACAACTCGAAGTCG-3' (SEQ ID N°5) and 5'-CGGTTCGACTCGCTTGTCG-3' (SEQ ID N°6).

The PCR reaction was carried out in a final volume of 50 $\mu$l consisting of 1X PCR buffer, 0.2 mM of each dNTP, 5 $\mu$M of each primer, 20 ng of template DNA and 1.25 U of Taq DNA polymerase (Taq DNA polymerase, ROCHE). Thirty cycles were performed for the amplification of template DNA. Initial denaturation was performed at 94°C for 4 min. Each subsequent cycle consisted of a 94°C (1 min) melting step, a 55°C (1 min) annealing step, and a 72°C (1 min) extension step. Samples obtained after the PCR reaction were run on agarose gel (1%) stained with ethidium bromide.

PCR amplification carried out with primers specific for the light chain revealed a single band at 348 bp for cells co-transfected with both plasmids (data not shown). Positive cells carrying the light chain sequence were also tested for the presence of the sequence coding for the heavy chain. Gel electrophoresis analysis performed on PCR product amplified using the primers specific for the heavy chain revealed a single band at 448 bp (data not shown). No band was detected in cells transformed with the control vector. These results validated the incorporation of both genes encoding for light and heavy chains in the genome of *Nannochloropsis oculata.*

f) Detection of fully-assembled cetuximab

The immunodetection of cetuximab was performed under non-reducing conditions. Ten $\mu$L of extra cellular medium from 6 positive clones were separated by SDS-PAGE using a 12% polyacrylamide gel. The separated proteins were transferred onto nitrocellulose membrane and stained with Ponceau Red in order to control transfer efficiency. The nitrocellulose membrane was blocked overnight in milk 5% dissolved in TBS for immunodetection. Immunodetection was then performed using horseradish peroxidase-conjugated goat anti-human IgG (SIGMA-ALDRICH, A6029) (1:2000 in TBS-T containing milk 1% for 1h30 at room temperature). Membranes were then washed with TBS-T (6 times, 5 minutes, room temperature) followed by a final wash with TBS (5 minutes, room temperature). Final development of the blots was performed by chemiluminescence method.

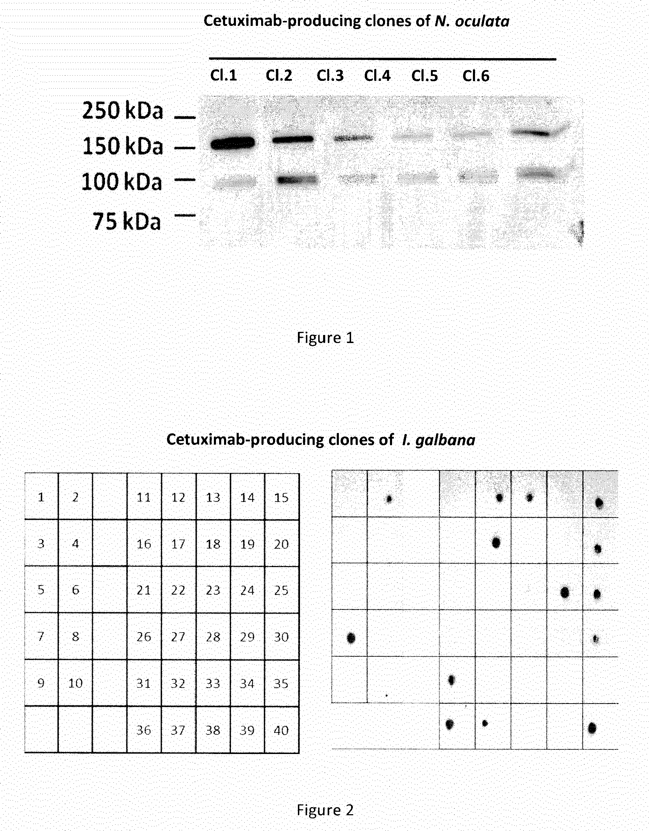

As depicted in figure 1, a major band at approximately 150 kDa was detected in the extracellular fraction of clones co-transfected with cetuximab light and heavy chains. This result is in agreement with a molecular weight of 152 kDa for cetuximab including carbohydrates as previously published (see Erbitux: EPAR Scientific Discussion available on the European Medicines Agency website). Differences in band intensity between clones were shown and corresponded to various level of expression of cetuximab. A second band at a lower molecular weight around 100 kDa was also detected which could correspond to non-fully assembled cetuximab.

The presence of light and heavy chains is assessed by SDS-PAGE electrophoresis under reducing condition. Proteins are detected using Coomassie blue staining.

g) Purification of cetuximab by affinity chromatography

The secreted cetuximab is purified by affinity chromatography method. Culture media of *N. oculata* at exponential phase of growth were collected from positive clones based on PCR analysis. Cells were separated from the culture medium by centrifugation (10 minutes, 2150 g, 20°C). The supernatant was supplemented with 1 mM PMSF and an equal volume of loading buffer (Glycine 1.5 M, NaCl 2.4 M, pH 9) was added before filtration using a membrane filter of 0.22 $\mu$m pore size. Sample was loaded onto a protein A-sepharose resin (HiTrap™ rProtein A FF, GE Healthcare) at a flow rate of 5-7 ml.min$^{-1}$ The column is washed with 4 column volumes of washing buffer (Glycine 1.5 M, NaCl 3 M, pH 9) at a similar flow rate. Elution was performed with 2 column volumes of Tris-Glycine buffer (Glycine 0.2 M, pH 2.5) at a flow rate of 0.5 ml.min$^{-1}$.

h) Deglycosylation assay

To detect the presence of glycans attached to the heavy chain of cetuximab, deglycosylation assay was performed on samples purified by affinity chromatography using peptide-*N*-glycosidase F (PNGase F, New England Biolabs) or endoglycosidase H (Endo H, New England Biolabs) according to manufacturer's recommendations. Digested samples were separated by SDS-PAGE using a 12% polyacrylamide gel. The separated proteins were transferred onto nitrocellulose membrane and stained with Ponceau Red in order to control transfer efficiency. The nitrocellulose membrane was blocked in TBS + 2% Tween-20. Affinodetection was performed using horseradish peroxidase-conjugated Concanavalin A (SIGMA-ALDRICH, L6397) by incubation with the lectin (1:1000) in TBS + 0.05% Tween-20 containing 1 mM $CaCl_2$, 1 mM $MnCl_2$ and 1 mM $MgCl_2$ for 2 hours at room temperature. After washing with TBS + 0.05% Tween (6 times, 5 minutes) and a final wash with TBS, binding of this lectin was detected by chemiluminescence method.

A band at approximately 55 kDa was detected in non-treated purified samples of clones co-transfected with cetuximab light and heavy chains (data not shown). This band corresponds to the heavy chain of cetuximab. Staining with Concanavalin A reveals the presence of N-glycans attached to the heavy chain of the cetuximab produced in *Nannochloropsis oculata.* On the contrary, no band was detected in purified samples treated by PNGase F. This result suggests the lack of fucose alpha 1,3-linked to the core region of the cetuximab heavy chain N-glycans.

i) Analysis of the cetuximab protein sequences

Fifteen $\mu$L of the purified cetuximab is separated by SDS-PAGE using a 12% polyacrylamide gel. Protein bands are stained with Coomassie brilliant blue CBB R-350 (Amersham Bioscience). The CBB-stained proteins on SDS-PAGE corresponding to the light and heavy chains of cetuximab are excised and digested with sequencing grade modified trypsin (Promega) or arginine-C (Princeton Separations). The gel piece is washed with 50% acetonitrile/0.1 M ammonium bicarbonate, and then dehydrated with acetonitrile. The protein in gel pieces is reduced with 10 mM dithiothreitol and alkylated with 55mM iodoacetamide. The gel piece is washed once with 20 mM ammonium bicarbonate and dehydrated with acetonitrile. The trypsin solution is added to the gel piece, and the enzyme reaction is allowed to proceed overnight at 37 °C. Alternatively, the arginine-C solution is added to the gel piece, and the enzyme reaction is allowed to proceed overnight at room temperature. Both supernatants from trypsin or arginine-C are acidified by adding trifluoroacetic acid and immediately subjected to mass spectrometry or stored in a freezer until analysis. Nano-LC/MS/MS experiments are performed on Q-TOF 2 and Ultima API hybrid mass spectrometers (Waters) equipped with a nano-electrospray ion source and a CapLC system (Waters). The mass spectrometers are operated in data-directed acquisition mode. For protein identification, all MS/MS spectra are searched using the SwissProt data-base.

j) Structural characterization of N-linked glycans of cetuximab

Heavy chain of cetuximab purified from the extracellular medium of *N, oculata* or available commercially (Erbitux®, Merck KGaA Darmstadt) is subjected to enzymatic deglycosylation using either peptide-N-glycosidase F (PNGase F, New England Biolabs) or endoglycosidase H (Endo H, New England Biolabs) in order to release N-linked glycans. Released glycans are analyzed by mass spectrometry as described by Dolashka et al. (2010) Glycan structures and antiviral effect of the structural subunit RvH2 of Rapana hemocyanin, Carbohyd Res., 345:2361-2367.

k) Binding characteritics of cetuximab

Binding characteristics of cetuximab purified from the extracellular medium of *N. oculaca* or available commercially (Erbitux®, Merck KGaA Darmstadt) are determined using flow cytometric analysis. Two EGFR expressing cancer cell lines sourced from the American Type Culture Collection are cultured and used for this analysis: HTB-132/MDA-MB-468 and CRL-1555/A431. EC50 values are determined from Competitive *binding* experiments as described in Keeler el al. (2004) Dual Mode of Action of a Human Anti-Epidermal Growth Factor Receptor Monoclonal Antibody for Cancer Therapy, J Immunol, 173:4699-4707.

l) Antibody-dependent cellular cytotoxicity (ADCC) of cetuximab

The ADCC activity of cetuximab purified from the extracellular medium of *N. oculata* or available commercially (Erbitux®, Merck KGaA Darmstadt) are determined by flow cytometric analysis using the single cell-based fluorogenic cytotoxicity kit GranToxiLux® PLUS (OncoImmunin, Inc.'s) following manufacturer's instruction. This experiment is realized for each EGFR expressing cancer cell lines described in example 1.m. Effector cells used for this experiment are PBMC purified from blood samples of human donors according to standard procedure using centrifugation on Ficoll density gradient.

Example 2 Secretion of the chimeric monoclonal antibody cetuximab in the culture medium of transformed *Isochrysis galbana*

To test the ability of *Isochrysis galbana* (*I. galbana)* to express a fully assembled monoclonal antibody that can be secreted into the extracellular medium, the co-transfection of the nuclear genome was carried out with 2 vectors, each containing either the light chain (SEQ ID N°1) or heavy chain (SEQ ID N°2) of cetuximab.

a) Standard culture conditions of *Isochrysis galbana*

Methods and conditions for culture of *I. galbana* were similar as those described for *Nannochloropsis oculala* in example 1.a.

b) Expression constructs for cetuximab expression

Two vectors, each containing light chain or heavy chain of cetuximab and sh ble gene, were used for co-transfection as described in example 1.b.

c) Genetic transformation

The co-transfection of *I. galbana* with vectors containing light and heavy chains of cetuximab was carried out by electroporation as described in example 1.c).

Cultures of *I. galbana* were harvested at mid-log phase and washed four times in 384 mM D-sorbitol. Cell concentration was adjusted to $1.10^8$ cells.ml$^{-1}$ in 384 mM D-sorbitol, and 100 $\mu$L of this cell suspension and 0.1-1 $\mu$g DNA (equal mix of plasmids containing the light and heavy chains or the empty vector) were used for each transformation. Electroporation was performed on the basis of the parameters used for *Nannochloropsis oculata* in example 1.c).

After electroporation, cells were immediately transferred to 10 mL fresh culture medium and incubated in low light overnight. Microalgae were placed in fresh medium containing 200$\mu$g.ml$^{-1}$ zeocin under standard conditions.

d) Polymerase chain reaction (PCR analysis

The incorporation of the heterologous chimeric light chain and heavy chain sequences in the genome of *Isochrysis galbana* was assessed by PCR analysis.

DNA extraction from zeocin-resistant polyclonal cultures was performed as described in example 1.d and amplification by PCR was carried out following the protocol described in example 1.e with the same primers.

PCR amplification carried out with primers specific for the light chain revealed a single band at 348 bp for cells co-transfected with both plasmids (data not shown). Positive cells carrying the light chain sequence were also tested for the presence of the sequence coding for the heavy chain. Gel electrophoresis analysis performed on PCR product amplified using the primers specific for the heavy chain revealed a single band at 448 bp (data not shown). No band was detected in cells transformed with the control vector. These results validated the incorporation of both genes encoding for light and heavy chains in the genome of *Isochrysis galbana.*

e) Detection of cetuximab in the extracellular medium of transformed *Isochrysis galbana*

Detection of cetuximab in the extracellular media of transformed *I. galbana* was performed by the dot blot method. Culture media of *I. galbana* at exponential phase of growth were collected from 38 positive polyclonal cultures based on PCR analysis and 2 wild type cultures. Cells were separated from the culture medium by centrifugation (10 minutes, 2150 g, 20°C).

Samples of extracellular media ($2\mu$L) were spotted onto a nitrocellulose membrane. The membrane was dried at room temperature and incubated for 1h in milk 5% dissolved in TBS. The membrane was rinsed for 5 min in TBS-T and then incubated with horseradish peroxidase-conjugated goat anti-human IgG (SIGMA-ALDRICH, A6029) (1:2000 in TBS-T containing milk 1% for 1h30 at room temperature). The membrane was then washed with TBS-T (6 times, 5 minutes, room temperature) followed by a final wash with TBS (5 minutes, room temperature). Final development of the dot blot was performed by chemiluminescence method.

As depicted in figure 2, dot blot signal of various intensities were detected for cetuximab-producing clones of *I. galbana.* Clones I and 11 corresponding to wild type cultures reveal no signal. Clones number 7, 15, 17, 24, 25, 31, 36, and 40 produced high level signal following the detection using anti-human IgG. Signal of mid-intensity were also detected for clones number 2, 12, 13, 20, 30, and 37. These results suggest the expression of various level of cetuximab in the extracellular medium of transformed cells of *I. galbana.*

f) Detection of cetuximab by gel electrophoresis

Purification of cetuximab by affinity chromatography is realized on extracellular media from positive clones detected by dot blot.

Protein electrophoresis under non-reducing condition are performed on purified samples using horseradish peroxidase-conjugated goat anti-human IgG as described in example 1.g. to detect fully-assembled cetuximab.

The presence of light and heavy chains is assessed by SDS-PAGE electrophoresis under reducing condition. Proteins are detected using Coomassie blue staining.

**Claims**

1. A transformed microalga comprising a nucleic acid sequence operatively linked to a promoter, wherein said nucleic acid sequence encodes an amino acid sequence comprising:

   (i) an heterologous signal peptide; and
   (ii) a therapeutic antibody, a functional fragment or a derivative thereof,

   said transformed microalga expressing the therapeutic antibody, functional fragment or derivative thereof secreted in the extracellular media, and
   said microalga being selected among green algae except Volvocales, and among red algae, chromalveolates, and euglenids.

**2.** A transformed microalga according to claim 2, wherein said microalga is selected among the division of Chlorophytes (except Volvocales), Rhodophytes, Dinoflagellates, Diatoms, Eustigmatophytes, Haptophytes and Euglenids, preferably among the genus *Tetraselmis, Porphyridium, Symbiodinium, Thalassiosira, Nannochloropsis, Emiliania, Pavlova, Isochrysis, Eutreptiella, Euglena,* most preferably among the genus *Nannochloropsis, Isochrysis* and *Tetraselmis,* and still most preferably among the species *Nannochloropsis oculata, Isochrysis galbana* and *Tetraselmis suecica.*

**3.** A transformed microalga according to any one of claims 1 to 2, wherein said therapeutic antibody, functional fragment or derivative thereof can be a human antibody, a chimeric antibody and/or a humanized antibody, a functional fragment or derivative thereof.

**4.** A transformed microalga according to any one of claims 1 to 3, wherein said therapeutic antibody, functional fragment or derivative thereof has an increased antibody-dependant cell-mediated cytotoxicity (ADCC), preferably an increase in ADCC of at least about 10%, 20%, 30%, 40%, 50%, 60%, 70%, 80%, 90%, 100%, 150%, 200%, 250%, 300%, 325%, 400%, or 500% in comparison to a control.

**5.** A transformed microalga according to any one of claims 1 to 4, wherein said therapeutic antibody, functional fragment or derivative thereof has a low fucose content, preferably said therapeutic antibody, functional fragment or derivative thereof contains less than 20%, preferably less than 15%, 10%, and even more preferably less than 5%, 1% or 0,1% of fucosylated oligosaccharides on its N-glycan structures.

**6.** A method for producing a therapeutic antibody, a functional fragment or a derivative thereof which is secreted in the extracellular medium, said method comprising the steps of:

(i) culturing a transformed microalga as defined in any one of claims 1 to 5;
(ii) harvesting the extracellular medium of said culture; and
(iii) purifying the therapeutic antibody, a functional fragment or a derivative thereof, which is secreted in said extracellular medium.

**7.** The method according to claim 6, wherein said method further comprises a step of determining :

■ the ADCC of said therapeutic antibody, functional fragment or derivative thereof, and/or
■ the glycosylation pattern of said therapeutic antibody, functional fragment or derivative thereof, and/or
■ the fucose content of said therapeutic antibody, functional fragment or derivative thereof.

**8.** The method according to any one of claims 6 to 7, wherein said method leads to the secretion of at least 25%, 30%, 35%, 40%, 45%, 50%, 55%, 60%, 65%, 70%, 75%, 80%, 85%, 90% or 90% of the therapeutic antibody, functional fragment or derivative thereof expressed in said microalgae.

**9.** Use of a transformed microalga according to any one of claims 1 to 5 for the production and the secretion of a therapeutic antibody, a functional fragment or a derivative thereof in the extracellular medium.

**10.** The use according to claim 9, wherein said therapeutic antibody, functional fragment or derivative thereof presents an increase antibody-dependant cell-mediated cytotoxicity (ADCC), preferably an increase in ADCC of at least about 10%, 20%, 30%, 40%, 50%, 60%, 70%, 80%, 90%, 100%, 150%, 200%, 250%, 300%, 325%, 400%, or 500% in comparison to a control.

**11.** The use according to any one of claims 9 to 10, wherein said therapeutic antibody, functional fragment or derivative thereof has a low fucose content, and even more preferably contains less than 10%, preferably less than 9%, 8%, 7%, 6% and even more preferably less than 5%, 1% or 0,1% of fucosylated oligosaccharides on its N-glycan structures.

**12.** A therapeutic antibody, a functional fragment or a derivative thereof produced and secreted in the extracellular medium of microalgae by a method according to any one of claims 6 to 8.

**13.** The therapeutic antibody, a functional fragment or a derivative thereof according to claim 12, wherein said therapeutic antibody, functional fragment or derivative thereof presents an increase antibody-dependant cell-mediated cytotoxicity (ADCC), preferably an increase in ADCC of at least about 10%, 20%, 30%, 40%, 50%, 60%, 70%, 80%, 90%, 100%, 150%, 200%, 250%, 300%, 325%, 400%, or 500% in comparison to a control.

14. The therapeutic antibody, a functional fragment or a derivative thereof according to any one of claims 12 to 13, wherein said therapeutic antibody, functional fragment or derivative thereof has a low fucose content has a low fucose content, and even more preferably contains less than 20%, preferably less than 15%, 10%, and even more preferably less than 5%, 1% or 0,1% of fucosylated oligosaccharides on its N-glycan structures.

15. A pharmaceutical composition comprising a therapeutic antibody, a functional fragment or a derivative thereof according to any one of claims 12 to 14.

**Cetuximab-producing clones of *N. oculata***

Figure 1

**Cetuximab-producing clones of *I. galbana***

Figure 2

| | Europäisches Patentamt European Patent Office Office européen des brevets | EUROPEAN SEARCH REPORT | Application Number EP 12 00 3136 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2004/046190 A2 (UNIV WARWICK [GB]; FRIGERIO LORENZO [IT]; HADLINGTON JANE [GB]) 3 June 2004 (2004-06-03) * page 6, paragraph 5; claim 1 * | 1-15 | INV. C12N15/82 |
| Y | WO 2011/090708 A2 (MERIAL LTD [US]; GUO XUAN [US]; TROUPE KAROLYN MARIE [US]; FEILMEIER B) 28 July 2011 (2011-07-28) * paragraphs [0036], [0112] - [0117]; claim 19; example 2 * | 1-11 | |
| X | GRIESBECK CHRISTOPH ET AL: "Chlamydomonas reinhardtii", MOLECULAR BIOTECHNOLOGY, vol. 34, no. 2, Sp. Iss. SI, October 2006 (2006-10), pages 213-223, XP002681386, ISSN: 1073-6085 | 12-15 | |
| Y | * table 3 * | 1-11 | |
| X | HEMPEL FRANZISKA ET AL: "Algae as Protein Factories: Expression of a Human Antibody and the Respective Antigen in the Diatom Phaeodactylum tricornutum", PLOS ONE, vol. 6, no. 12, December 2011 (2011-12), XP002681385, ISSN: 1932-6203 | 12-15 | TECHNICAL FIELDS SEARCHED (IPC) C12N |
| A | * the whole document * | 1-11 | |

-/--

The present search report has been drawn up for all claims

| Place of search | Date of completion of the search | Examiner |
|---|---|---|
| Munich | 9 August 2012 | Krüger, Julia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt
European
Patent Office
Office européen
des brevets

# EUROPEAN SEARCH REPORT

**Application Number**

EP 12 00 3136

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YANGMIN GONG ET AL: "Microalgae as platforms for production of recombinant proteins and valuable compounds: progress and prospects", JOURNAL OF INDUSTRIAL MICROBIOLOGY & BIOTECHNOLOGY ; OFFICIAL JOURNAL OF THE SOCIETY FOR INDUSTRIAL MICROBIOLOGY, SPRINGER, BERLIN, DE, vol. 38, no. 12, 1 September 2011 (2011-09-01), pages 1879-1890, XP019982203, ISSN: 1476-5535, DOI: 10.1007/S10295-011-1032-6 | 12-15 | |
| A | * the whole document * * page 1881, column 2 * ----- | 1-11 | |
| A | WO 2012/052170 A1 (ALGENICS [FR]; LEJEUNE ALEXANDRE [FR]; MICHEL REMY [FR]; CADORET JEAN-) 26 April 2012 (2012-04-26) * page 1 * * page 7, paragraph 3 * * page 12, line 20; claim 1 * ----- | 1-11 | |
| A | FRANKLIN SCOTT E ET AL: "Recent developments in the production of human therapeutic proteins in eukaryotic algae", EXPERT OPINION ON BIOLOGICAL THERAPY, INFORMA HEALTHCARE, UK, vol. 5, no. 2, 1 February 2005 (2005-02-01), pages 225-235, XP009161265, ISSN: 1744-7682 ----- -/-- | 1-15 | **TECHNICAL FIELDS SEARCHED (IPC)** |

The present search report has been drawn up for all claims

| Place of search | Date of completion of the search | Examiner |
|---|---|---|
| Munich | 9 August 2012 | Krüger, Julia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 2 660 324 A1

## EUROPEAN SEARCH REPORT

Europäisches Patentamt
European Patent Office
Office européen des brevets

Application Number

EP 12 00 3136

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MAMEDOV T ET AL: "Green algae Chlamydomonas reinhardtii possess endogenous sialylated N-glycans", FEBS OPEN BIO 2011 ELSEVIER NLD LNKD-DOI:10.1016/J.FOB.2011.10.003, vol. 1, December 2011 (2011-12), pages 15-22, XP002681539, ISSN: 2211-5463 * page 21, column 1, paragraph 2 * ----- | 1-15 | |

TECHNICAL FIELDS
SEARCHED      (IPC)

The present search report has been drawn up for all claims

| Place of search | Date of completion of the search | Examiner |
|---|---|---|
| Munich | 9 August 2012 | Krüger, Julia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 12 00 3136

This annex lists the patent family members relating to the patent documents cited in the above-mentioned European search report.
The members are as contained in the European Patent Office EDP file on
The European Patent Office is in no way liable for these particulars which are merely given for the purpose of information.

09-08-2012

| Patent document cited in search report | | Publication date | Patent family member(s) | | Publication date |
|---|---|---|---|---|---|
| WO 2004046190 | A2 | 03-06-2004 | AU | 2003302026 A1 | 15-06-2004 |
| | | | CA | 2506505 A1 | 03-06-2004 |
| | | | EP | 1578800 A2 | 28-09-2005 |
| | | | US | 2006276637 A1 | 07-12-2006 |
| | | | US | 2011162113 A1 | 30-06-2011 |
| | | | WO | 2004046190 A2 | 03-06-2004 |
| WO 2011090708 | A2 | 28-07-2011 | AR | 079767 A1 | 15-02-2012 |
| | | | US | 2011162115 A1 | 30-06-2011 |
| | | | WO | 2011090708 A2 | 28-07-2011 |
| WO 2012052170 | A1 | 26-04-2012 | EP | 2444495 A1 | 25-04-2012 |
| | | | WO | 2012052170 A1 | 26-04-2012 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2003091413 A
- WO 2009064777 A
- US 5225539 A
- GB 2188638 A
- US 5585089 A

**Non-patent literature cited in the description**

- **WALSH.** Biopharmaceutical benchmarks. *Nature Biotech.,* 2010, vol. 28, 917-924
- **PAUL ; MA.** Plant-made pharmaceuticals: Leading products and production platforms. *Biotechnol. Appl. Biochem.,* 2011, vol. 58, 58-67
- **SPOLAORE et al.** Commercial applications of microalgae. *J. Biosci. Bioeng.,* 2006, vol. 101, 87-96
- **RASALA ; MAYFIELD.** The microalga Chlamydomonas reinhardtii as a platform for the production of human protein therapeutics. *Bioeng. Bugs,* 2011, vol. 2, 50-54
- **BECK et al.** Trends in glycosylation, glycoanalysis and glycoengineering of therapeutic antibodies and Fc-fusion proteins. *Current Pharm. Biotechnol.,* 2008, vol. 9, 482-501
- **HEMPEL et al.** *PLos ONE,* 2011, vol. 6 (12), 28424
- **ZAVLASKAÏA ; LIPPMEIER.** Transformation of the diatom Phaeodactylum tricornutum (Bacillariophyccac) with a variety of selectable marker and reporter genes. *J. Phycol.,* 2000, vol. 36, 379-386
- **KILIAN et al.** High-efficiency homologous recombination in the oil-producing alga Nannochloropsis. *Proc. Natl. Acad. Sci.USA,* 2011, vol. 108, 21265-21269
- **VON HEIJNE.** The signal Peptide. *J. Membr. Biol.,* 1990, vol. 115, 195-201
- **EMANUELSSON et al.** Locating proteins in the cell using TargetP, SignalP and related tools. *Nat. Protoc.,* 2007, vol. 2, 953-971
- **VERNET ; SCHATZ.** Protein translocation across membranes. *Science,* 1988, vol. 241, 1307-1313
- **PETERSEN et al.** SignalP 4.0: discriminating signal peptides from transmembrane regions. *Nat. Methods,* 2011, vol. 8, 785-786
- **KABAT.** Sequences of Proteins of Immunological Interest. National Institutes of Health, 1987

- **CHOTHIA ; LESK.** Canonical structures for the hypervariable regions of immunoglobulins. *J. Mol. Biol.,* 1987, vol. 196, 901-17
- **CHOTHIA et al.** Conformations of immunoglobulin hypervariable regions. *Nature,* 1989, vol. 342, 878-83
- **RIECHMANN et al.** Reshaping human antibodies for therapy. *Nature,* 1988, vol. 332, 323-327
- **SHITARA et al.** Chimeric antiganglioside GM2 antibody with antitumor activity. *Cancer Immunol. Immunother,* 1993, vol. 36, 373-380
- **SÉVENO et al.** Plant N-glycan profiling of minute amounts of material. *Anal. Biochem.,* 2008, vol. 379, 66-72
- **STADLMANN et al.** Analysis of immunoglobulin glycosylation by LC-ESI-MS of glycopeptides and oligosaccharides. *Proteomics,* 2008, vol. 8, 2858-2871
- **BARDOR et al.** Monoclonal C5-1 antibody produced in transgenic alfalfa plants exhibits a N-glycosylation that is homogenous and suitable for glyco-engineering into human-compatible structures. *Plant Biotechnol. J.,* 2003, vol. 1, 451-462
- **JENSEN et al.** Cell-associated degradation affects the yield of secreted engineered and heterologous proteins in the Bacillus subtilis expression system. *Microbiology,* 2000, vol. 146, 2583-2594
- **KILIAN.** High-efficiency homologous recombination in the oil-producing alga. *Nannochloropsis sp.,* 2011, vol. 108, 21265-21269
- **DOLASHKA et al.** Glycan structures and antiviral effect of the structural subunit RvH2 of Rapana hemocyanin. *Carbohydr Res.,* 2010, vol. 345, 2361-2367
- **KEELER.** Dual Mode of Action of a Human Anti-Epidermal Growth Factor Receptor Monoclonal Antibody for Cancer Therapy. *J Immunol,* 2004, vol. 173, 4699-4707